# EUROPEAN PATENT SPECIFICATION

(11) **EP 0 407 411 B1**
(45) Date of publication and mention of the grant of the patent: **10.11.1993**
(21) Application number: 89903330.2
(22) Date of filing: 01.03.1989
(51) Int. Cl.: C12N 15/67

(54) **ACTIVITION OF NF-kB PRECURSOR**
AKTIVIERUNG DER NF-KAPPA B-VORSTUFE
ACTIVATION DU PRECURSEUR NF-kB

(30) Priority: 01.03.1988 US 162680
(43) Date of publication of application: 16.01.1991
(73) Proprietor: WHITEHEAD INSTITUTE FOR BIOMEDICAL RESEARCH, Cambridge, MA 02142 (US)
(72) Inventor: BAEUERLE, Patrick, A., D-8033 Martinsried, (DE); BALTIMORE, David, Cambridge, MA 02138 (US)
(74) Representative: Holdcroft, James Gerald, Dr.
(86) International application number: US8900820
(87) International publication number: WO8908147

(56) References cited:
- Cell, volume 47, 26 December 1986, Cell Press, R. Sen et al "Inducibility of kappa immunoglobulin enhancer-binding protein NF-KappaB by a posttranslational mechanism", pages 921-928
- Proceedings of the National Academy of Sciences of the USA, volume 83, January 1986, R, Wall et al "A labile inhibitor blocks immunoglobulin Kappa-light-chain-gene transcription in a pre-B leukemic cell line", pages 295-298
- The Control of Human Retrovirus Gene Expression, Banbury Conference, Cold Spring Harbor, New York, volume 17, 1988, Franza et al (eds), P.A. Baeuerle et al "Activation of NF-KappaB : a transcription factor controlling expression of the immunoglobulin Kappa light-chain gene of HIV", pages 217-226
- Cell, volume 53, 22 April 1988, Cell Press, P.A. Baeuerle et al "Activation of DNA-binding activity in an apparently cytoplasmic precursor of the NF-KappaB transcription factor", pages 211-217

## Description

### Background

Enhancer and promoter elements are the most important regulatory DNA sequences known to control eucaryotic gene expression. McKnight, S.L. and R. Tjian, Cell, 46:795-805 (1986).

Promoter elements are restricted to a local region upstream of the gene. Enhancer elements are cis-acting sequences that display a great positional flexibility. Banerji, J. et al., Cell, 27:299-308 (1981), Fromm, M. and P. Beig, Molecular Cellular Biology, 3:991-999 (1983).

Enhancer elements are responsible for many regulatory properties of the genes they control, such as cell type and tissue - specific expression and responsiveness to steroid hormones and growth factors. All enhancer elements analyzed so far are composed of multiple DNA sequence motifs that serve as binding sites for specific protein factors. There is increasing evidence that sequence-specific DNA-binding proteins confer transcriptional activity and specialized regulatory properties to enhancer elements. Schoeler, H. and P. Gruss, EMBO Journal, 4:3005-3013 (1985); Ephrussi, A. et al., Science, 227:134-140 (1985); Lee, W. et al., Cell, 49:741-752 (1987). How enhancer elements control the rate of transcription from a particular gene is not yet understood.

A typical enhancer element is located in the J-C intron of the kappa light chain gene. Queen, C. and D. Baltimore, Cell, 33:741-748 (1983); Falkner, F.G. and H.G. Zachau, Nature, 310:71-74 (1984); Picard, D. and K.R. Yamamoto_{,} EMBO Journal, 6:3333-3340 (1987). This enhancer contains at least three sites that interact with specific DNA-binding proteins. Sen, R. and D. Baltimore, Cell, 46:705-716 (1986); Lenardo, M. et al., Science, 236:1573-1577 (1987). One of these sites, the kappaB site, binds a B-cell specific factor called nuclear factor kappaB (NF-kB). Sen, R. and D. Baltimore, Cell, 47:921-928 (1986); Sen, R. and D. Baltimore, Cell, 47:921-928 (1986). The DNA-binding activity of this protein is not detectable in nuclear extracts of the pre-B cell line 70Z/3, but can be activated by a post-translational mechanism after treatment of cells with bacterial lipopolysaccharide or phorbol esters. Sen, R. and D. Baltimore, Cell, 47:921-928 (1986). The induction of NF-kB activity in pre-B cells by these treatments strongly correlates with the transcriptional activity of the kappa gene. Nelson, K. J. et al., Proceedings of the National Academy of Sciences, USA, 82:5305-5309 (1985); Sen, R. and D. Baltimore, Cell, 47:921-928 (1986). Mutational analysis of the kappa enhancer (Lenardo, M. et al., Science, 236:1573-1577 (1987) and the construction of functional enhancers using oligonucleotides representing NF-kB binding sites have provided further evidence that the binding of NF-kB to the kB motif in the kappa enhancer is essential for the transcriptional activity, inducibility and developmental stage specificity of the kappa enhancer. NF-κB appears also to play a key role in the transcriptional activation of the human immunodeficiency virus (HIV) in latently infected T-cells because induction of NF-κB activity in T-cells dramatically increases the activity of the transcriptional control elements of the virus and mutation of the κB sites abolishes the stimulation. Nabel, G and D. Baltimore, Nature, 326:711-713 (1987).

### Summary of the Invention

The present invention relates to activation of NF-κB, a transcription factor which controls expression of the immunoglobulin kappa light chain gene and of human immunodeficiency virus (HIV), as well as to methods of causing and preventing activation of the factor, controlling expression of the immunoglobulin kappa light chain gene and controlling expression of HIV. It relates to a precursor of NF-κB which has been shown to be present in a variety of cells; control of induction of activity of the precursor; a protein inhibitor (I kappa B or IκB) that can convert NF-κB into an inactive form; methods by which the NF-κB precursor can be converted to active NF-κB; and methods by which initial expression of dormant HIV-DNA by latently-infected T-lymphocytes can be controlled.

More specifically, the present invention provides a method of altering expression in a cell of a gene whose transcriptional activity is altered by binding of nuclear factor kappa B (NF-κB) to the enhancer of said gene, comprising introducing an agent which controls dissociation of the nuclear factor kappa B-inhibitor of nuclear factor kappa B (NF-κB--IκB) complex present in the cytoplasm of said cell.

The invention also provides a method of reducing expression in a cell of a gene whose transcriptional activity is activated by binding of NF-κB to the enhancer of said gene, comprising introducing an agent which prevents dissociation of NF-κB--IκB complex present in the cytoplasm of said cell.

Further, the invention provides a method of activating in a host cell an NF-κB precursor present in the cytoplasm of said host cell, the precursor comprising an NF-κB--IκB complex, comprising contacting the host cell with an agent capable of causing dissociation of the complex into inhibitor of nuclear factor kappa B (IκB) and NF-κB and translocation of said NF-κB into the nucleus of said cell.

Still further, the present invention provides a method of controlling expression of human immunodeficiency virus DNA in a host cell latently infected with human immunodeficiency virus DNA, comprising preventing binding of NF-κB to human immunodeficiency virus transcriptional control elements.

The invention also provides NF-κB-IκB the subject of claims 8-12 hereof, IκB the subject of claims 13 and 14 and inactive cytosolic NF-κB precursor the subject of claim 15 hereof.

The methods of the present invention are useful in determining/controlling expression of the immunoglobulin kappa light chain gene, expression of HIV-DNA by latently-infected T-cells and expression of additional genes for which NF-κB plays a role as an enhancer-binding protein which participates in transcriptional activation of the gene. As a result, it is possible, for example to enhance expression of the immunoglobulin kappa light chain gene or to inhibit expression of HIV-DNA in latently infected cells. This is effected by activation of an NF-kB precursor shown to be present in the cytosolic cellular fraction.

### Brief Description of the Drawings

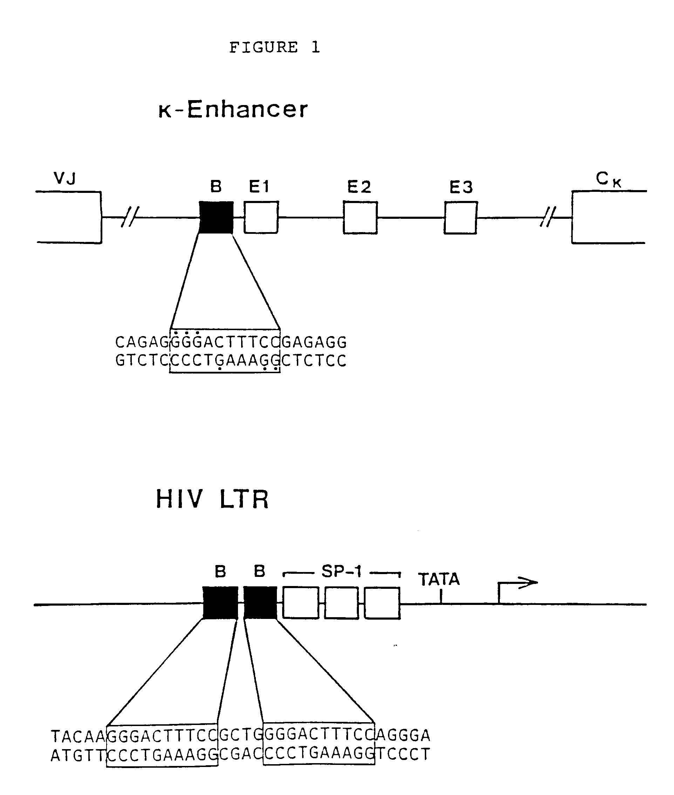

Figure 1 is a representation of binding sites for the NF-kB transcription factor in the immunoglobulin kappa light chain enhancer and the HIV enhancer. Boxes indicate the binding sites for NF-kB (B); other regulatory sites are referred to as E1, E2 and E3 and Spl. Dots indicate guanosine residues in the kappa enhancer whose methylation interfered with binding of NF-kB.

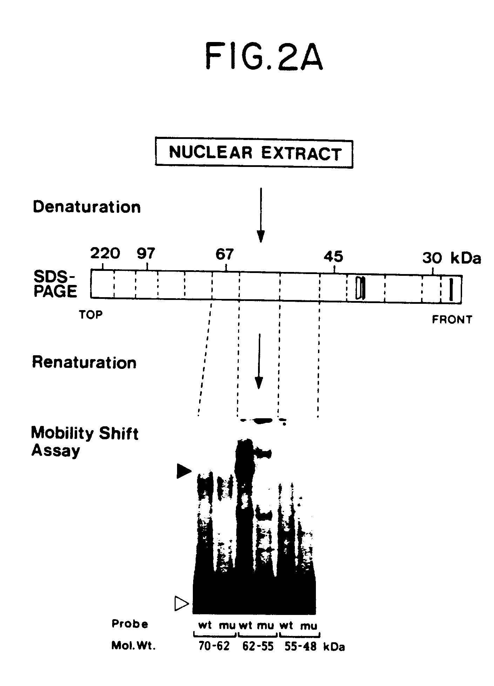

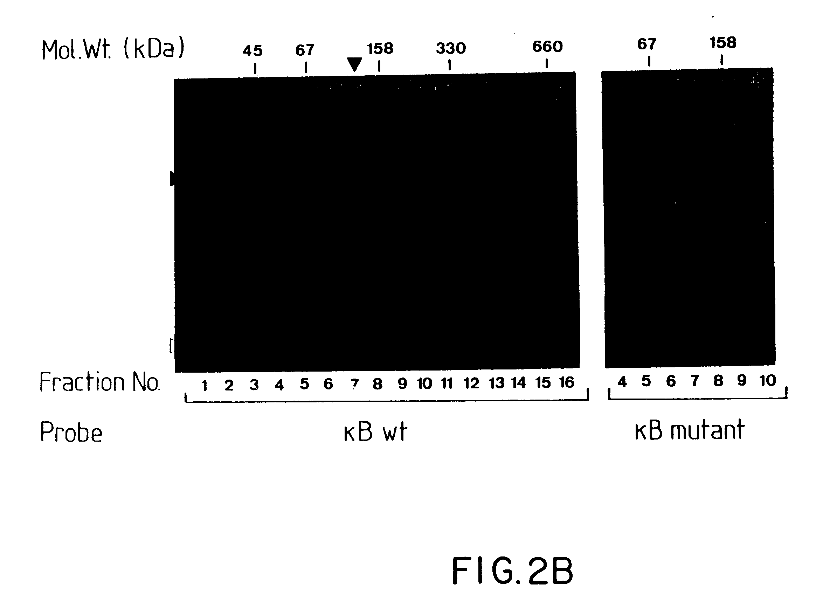

Figure 2 is a characterization of the NF-kB protein. Figure 2A represents determination of the molecular weight of NF-kB. Nuclear extract (300 ug of protein) from TPA-stimulated 70Z/3 pre-B cells was denatured and subjected to reducing SDS-polyacrylamide gel electrophoresis (SDS-PAGE). Protein in the molecular weight fractions indicated by dashed lines was eluted and renatured prior to mobility shift as says as described. A fluorogram of a native gel is shown. The filled arrowhead indicates the position of a specific protein DNA-complex only detected in the 62-55 kDa fraction with a wild type (wt) but not with a mutant (mu) kappa enhancer fragment. The open arrowhead indicates the position of unbound DNA-fragments. Figure 2B is a representation of glycerol gradient centrifugation of NF-kB. Nuclear extract (400 ug of protein) from TPA-stimulated 70Z/3 cells was subjected to ultracentrifugation on a continuous 10-30% glycerol gradient for 20 hours at 150,000xg in buffer D(+). Co-sedimented molecular weight standards (ovalbumin, 45 kDa; bovine serum albumin, 67 kDa; immunoglobulin G, 158 kDa; thyroglobulin monomer, 330 kDa and dimer 660 kDa) were detected in the fractions by SDS-PAGE, followed by Coomassie Blue staining. The distribution of NF-kB activity was determined by electrophoretic mobility shift assays using an end-labelled kappa enhancer fragment. Fluorograms of native gels are shown. The specificity of binding was tested using a kappa enhancer fragment with a mutation in the NF-kB binding site.

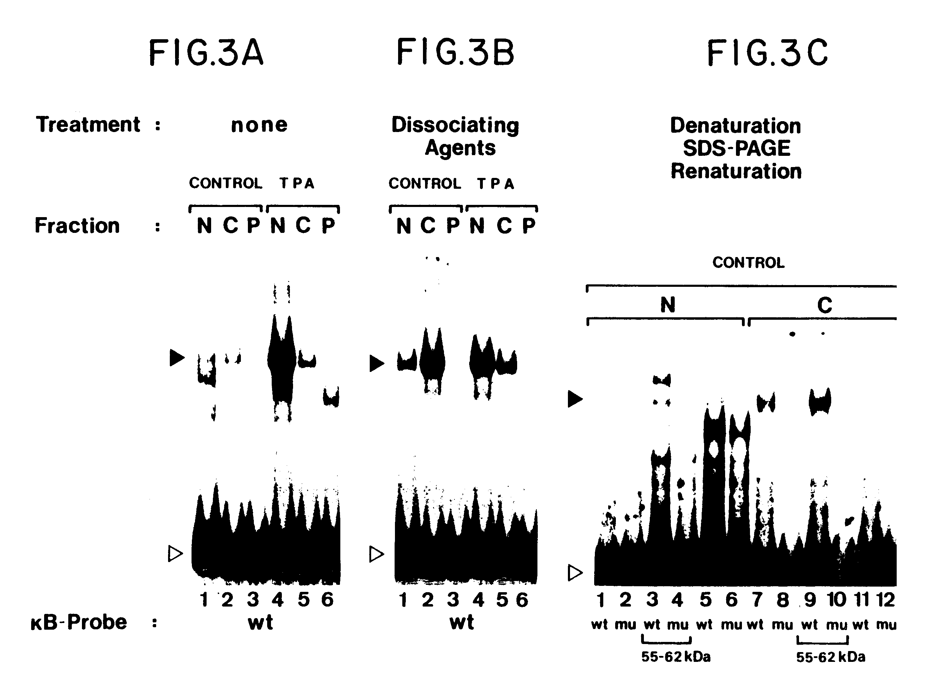

Figure 3 represents detection of a cytosolic precursor of NF-kB. Figure 3A represents analysis of subcellular fractions for NF-kB DNA-binding activity Nuclear extracts (N), cytosolic (C) and postnuclear membrane fractions (P) from control and TPA-stimulated 70Z/3 pre-B cells were analyzed by gel-shift assays. The filled arrowhead indicates the position of the specific protein-DNA complex seen only with a wild type but not with a mutant kappa enhancer fragment. Figure 3B represents activation of a cytosolic NF-kB precursor after treatment with dissociating agents. Subcellular fractions were treated with 25% formamide followed by dilution and addition of 0.2% sodium desoxycholate as described. Figure 3C represents detection of a cytosolic NF-kB precursor after denaturation, SDS-PAGE and renaturation of protein. Nuclear extract (N) and cytosolic fraction (C) from unstimulated (control) 70Z/3 cells was subjected to the treatment outlined in Figure 2A. For details of illustration, see Figure 3A.

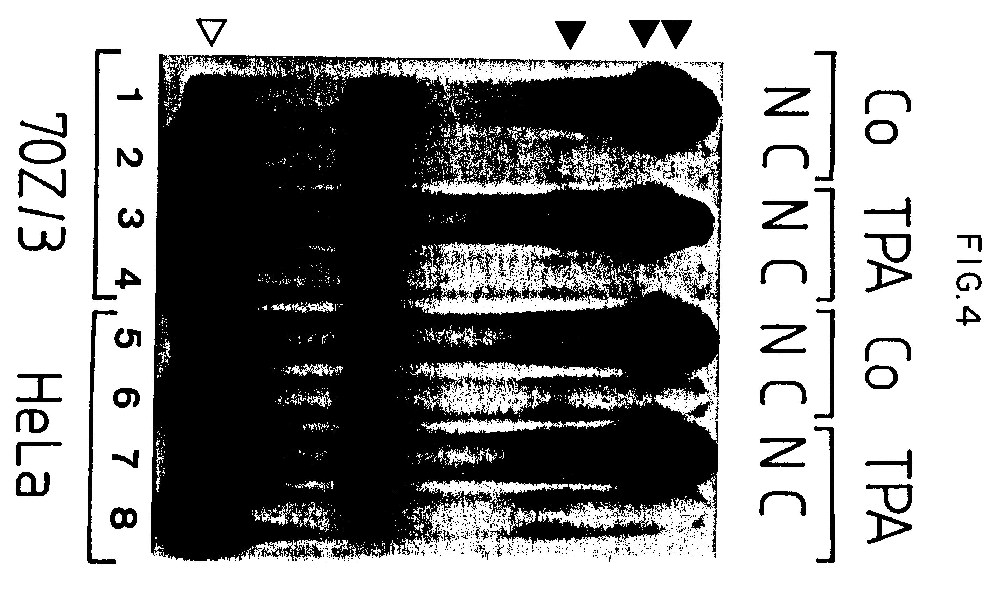

Figure 4 represents analysis of subcellular fractions for DNA-binding activity of the TPA-inducible transcription factor AP-1. Equal cell-equivalents of nuclear extracts (N) and cytosolic fractions (C) from 70Z/3 and HeLa cells were used in mobility shift assays. AP-1 specific DNA-binding activity was detected using an end-labeled EcoRI-HindIII fragment from the yeast HIS 4 promoter containing three binding sites for GCN4 recognized by mammalian AP-1. The three protein-DNA complexes seen on shorter exposures of the fluorogram are indicated by filled arrowheads and the position of unbound DNA-fragment by an open arrowhead.

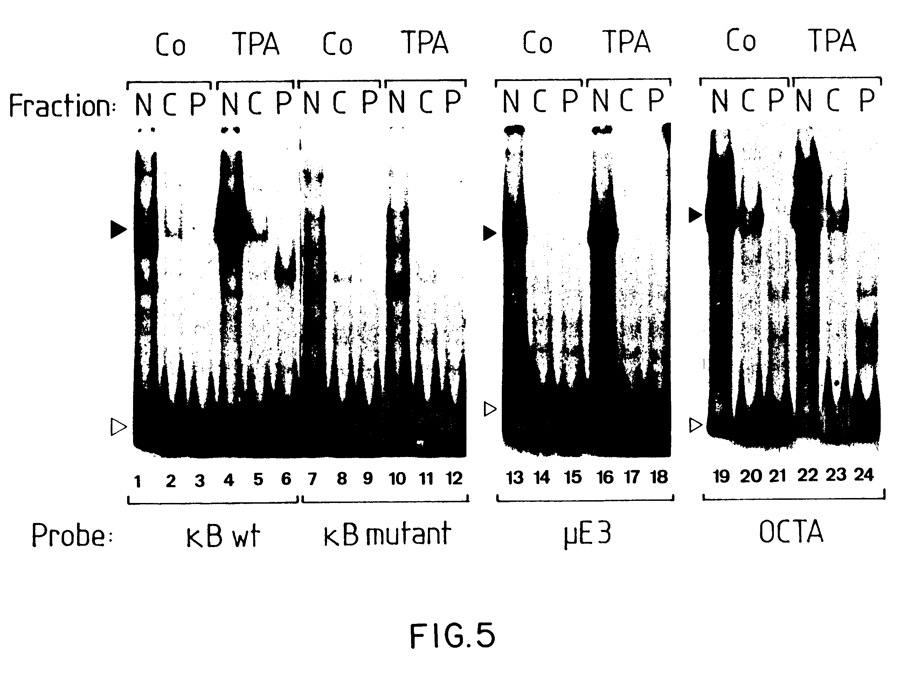

Figure 5 represents results of electrophoretic mobility shift analysis of subcellular fraction of 70Z/3 cells.

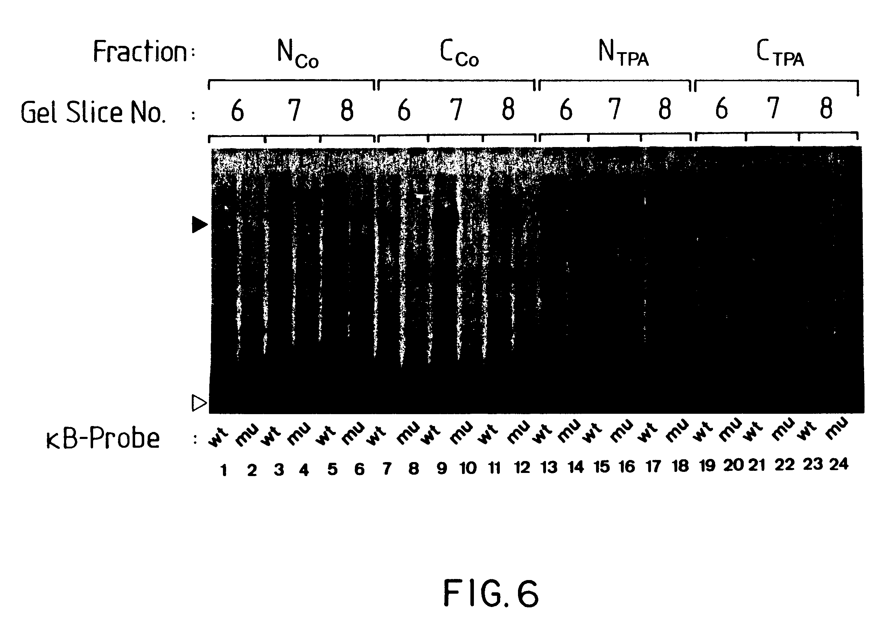

Figure 6 shows the effect of denaturation and renaturation of kB-specific DNA-binding activity in nuclear extracts and cytosolic fractions of 70Z/3 cells.

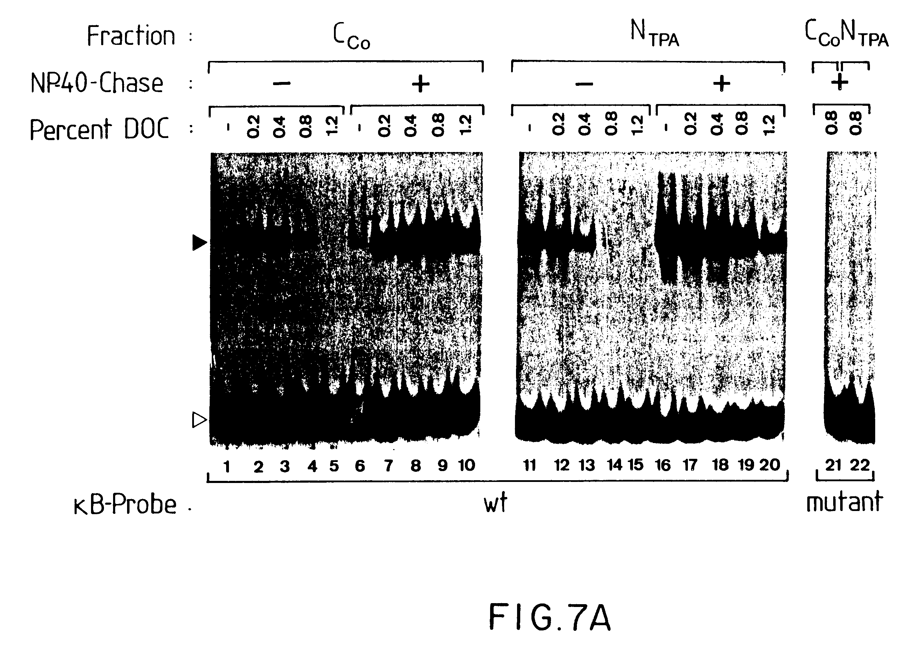

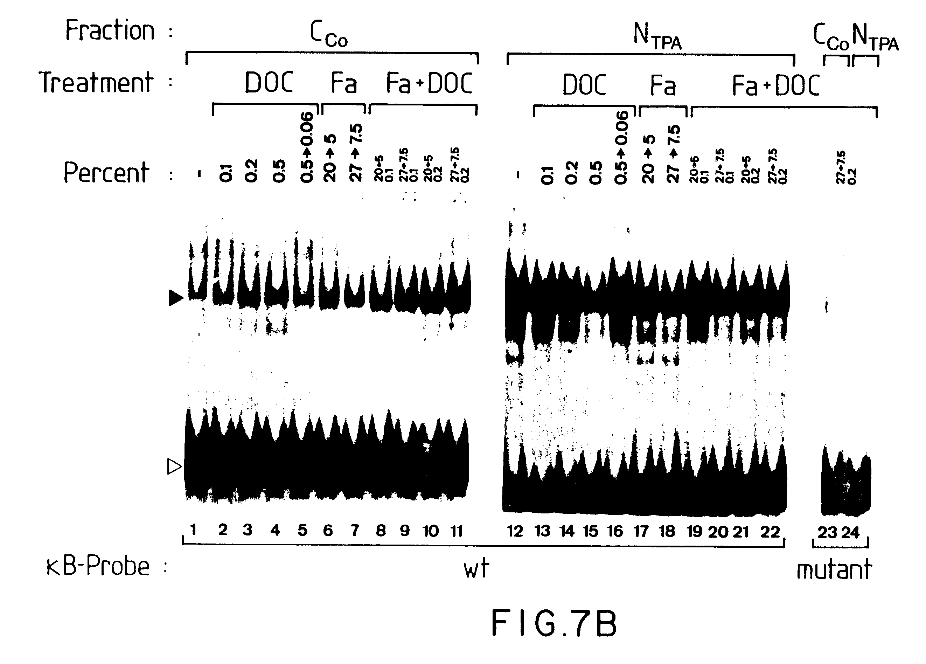

Figure 7 shows the effects of dissociating agents on the activity of NF-kB in subcellular fractions of 70Z/3 cells. Figure 7A: cell-free activation of a NF-kB precursor in the cytosolic fraction by desoxycholate. Figure 7B: cell-free activation of a NF-kB precursor in the cytosolic fraction by formamide and by a combined treatment with formamide and desoxycholate.

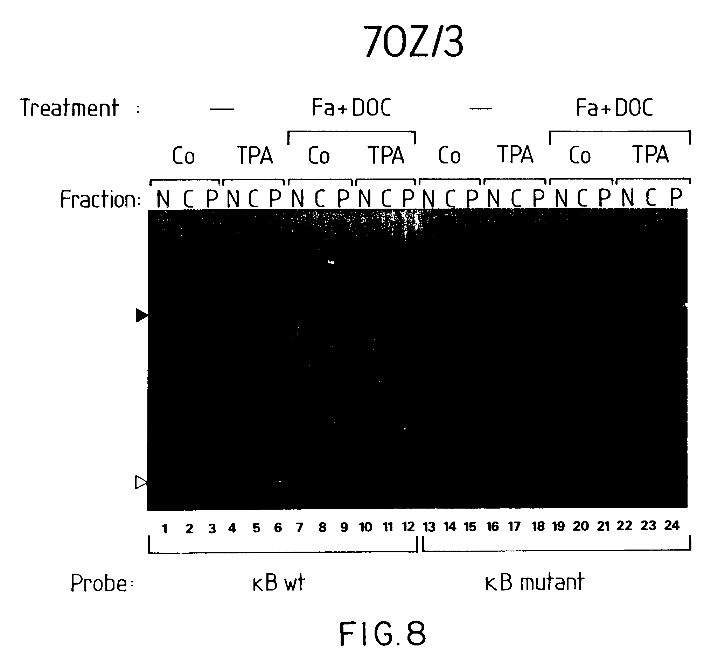

Figure 8 shows the effect of TPA stimulation on the subcellular distribution of NF-kB in 70Z/3 cells.

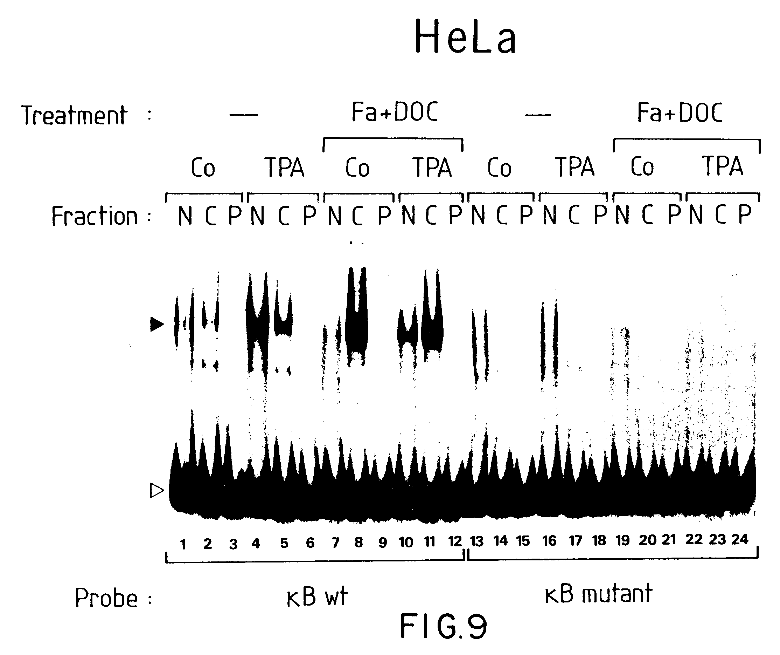

Figure 9 shows the effect of TPA stimulation on the subcellular distribution of NF-kB in HeLa cells.

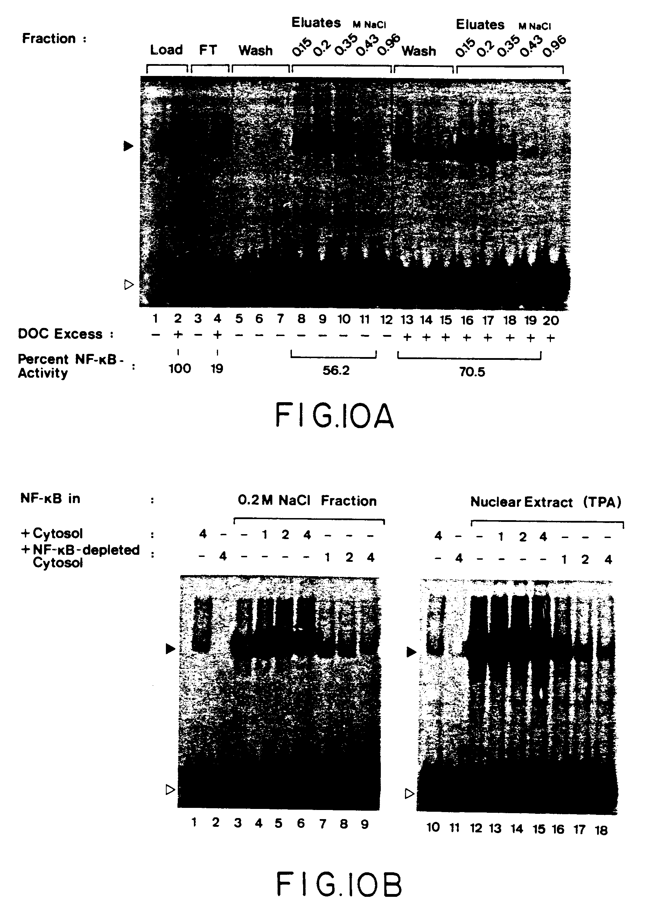

Figure 10 shows results of DNA-cellulose chromatography of DOC-treated cytosol. Cytosol was prepared from unstimulated 70Z/3 pre-B cells and protein concentrations determined. In the fluorograms of native gels shown, the filled arrowheads indicate the position of the NF-kB-k enhancer fragment complex and the open arrowheads the position of unbound DNA probe. Figure 10A: Release of DOC-independent NF-kB activity. Equal proportions of load, flow-through (FT), washings, and eluates were analyzed by EMSA, with (+) or without (-) excess DOC. The ³²P-radioactivity in the NF-kB-DNA complexes was counted by liquid scintillation and the percentage of NF-kB activity recovered in the various fractions was calculated. Figure 10B: Release of an inhibitory activity. NF-kB contained in the 0.2M NaCl fraction (31 ng of protein) or NF-kB in a nuclear extract from TPA-treated 70Z/3 cells (1.1 µg of protein) was incubated under non dissociating conditions with the indicated amounts (in microliters) of either cytosol which was DOC-treated but not passed over DNA-cellulose (lanes 4 to 6 and 13 to 15) or the flow-through fraction (referred to as NF-kB-depleted cytosol; lanes 7 to 9 and 16 to 18).

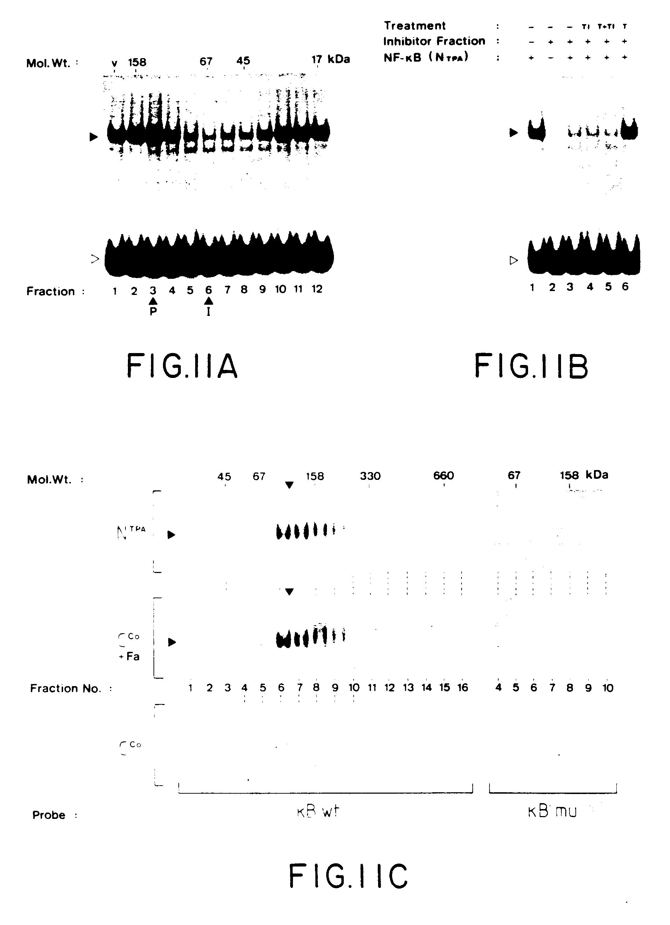

Figure 11 shows characterization of IkB and its complex with NF-kB. In the fluorograms shown, the filled arrowheads indicate the position of the NF-kB-DNA complex and the open arrowheads the position of free DNA probe. Figure 11A: For size determination of IkB, the flow-through from the DNA-cellulose column was passed over a G-200 Sephadex column. Portions of fractions were incubated with NF-kB contained in nuclear extracts from TPA-stimulated 70Z/3 cells (N TPA) and analyzed by EMSA. v, void volume; P, fraction where remaining NF-kB precursor (Figure 10A, lane 4) peaked after gel filtration as assayed with excess DOC in the absence of added NF-kB; I, fraction where the inhibiting activity peaked. Figure 11B: The effect of trypsin treatment on the inhibiting activity of IkB. NF-kB in a nuclear extract (lane 1) was incubated with a fraction containing inhibitor (lane 2) without any addition (-; lane 3) or with bovine pancreas trypsin inhibitor (TI; lane 4), trypsin that had been incubated with BPTI (T+TI; lane 5), or with trypsin alone (T; lane 6). Samples were then used in the inhibitor assay. Figure 11C: Glycerol gradient sedimentation of NF-kB and its complex with IkB. Nuclear extract from TPA-stimulated 70Z/3 cells (N TPA) and cytosol from unstimulated cells (C Co) were subjected to sedimentation through a glycerol gradient. Cosedimented size markers were ovalbumin (45 kD) , BSA (67 kD), immunoglobulin G (158 kD) and thyroglobulin (330 and 660 kD). NF-kB activity was detected in the fractions by EMSA with a wild type k enhancer fragment (kB wt, left panels). The specificity was tested with a mutant fragment (kB mu, right panels). The inactive cytosolic NF- κB precursor (lower panel) was activated by formamide treatment (Fa; middle panel).

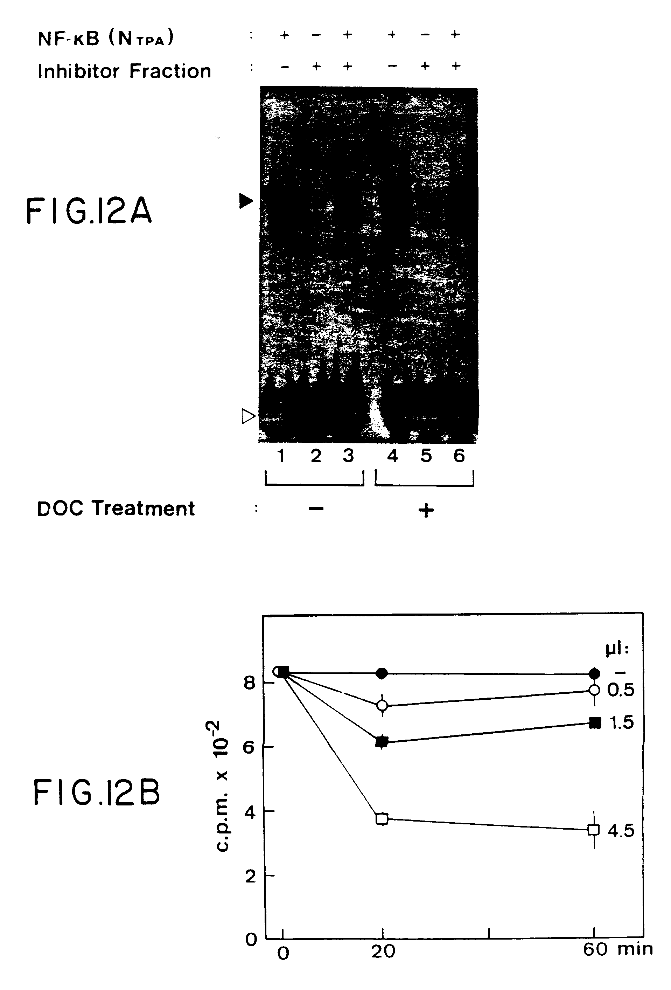

Figure 12 shows the reversibility and kinetics of the inactivation of NF-kB. Figure 12A: The effect of DOC treatment on in vitro inactivated NF-kB. NF-kB contained in nuclear extracts from TPA-stimulated 70Z/3 cells (N TPA; 1.1 µg of protein) was inactivated by addition of a gel filtration fraction containing IkB (2.5 µg of protein) . A duplicate sample was treated after the inhibition reaction with 0.8% DOC followed by addition.of DNA binding reaction mixture containing 0.7% NP-40®. Samples were analyzed by EMSA. In the fluorograms shown, the filled arrowhead indicates the position of the NF-kB-DNA complex and the open arrowhead the position of unbound DNA probe. Figure 12B: A titration and kinetic analysis of the in vitro inactivation of NF-kB. NF-kB contained in nuclear extracts from TPA- treated 70Z/3 cells (2.2 µg of protein) was incubated with increasing amounts (0.25 to 2.25 µg of protein) of a gel filtration fraction containing IkB. After the DNA binding reaction, samples were analyzed by EMSA. The ³²P-radioactivity in the NF-kB-DNA complexes visualized by fluorography was determined by liquid scintillation counting. All reactions were performed in triplicates. The bars represent standard deviations.

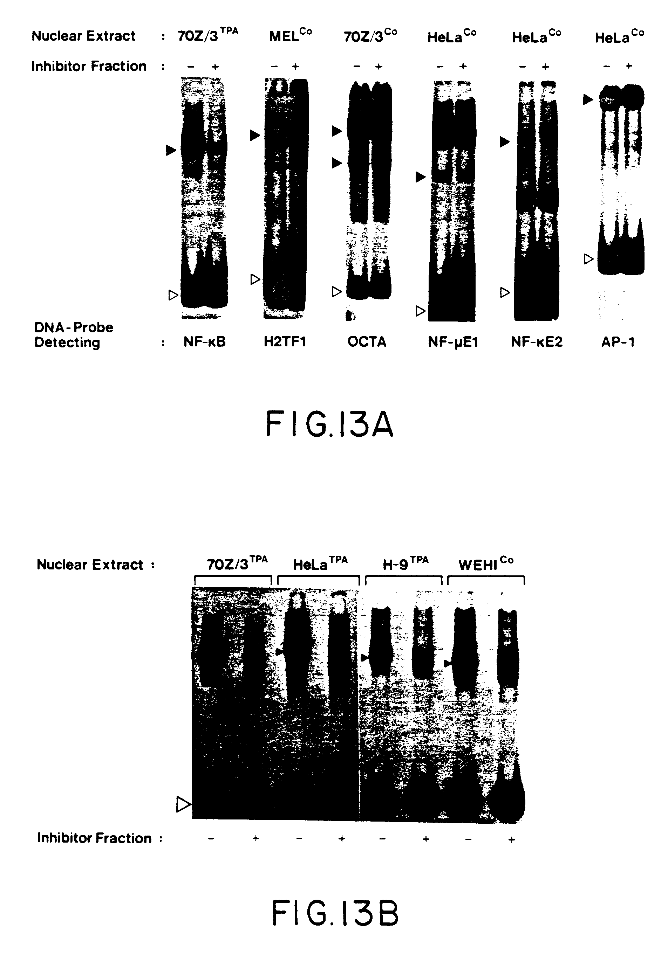

Figure 13 shows the specificity of IkB. Nuclear extracts from unstimulated (Co) or TPA-treated cells were incubated with 5 µl of buffer G (-) or with 5 µl of a gel filtration fraction containing IkB (+) (A, in the presence of 150 mM NaCl). After DNA binding reactions, samples were analyzed by EMSA. Figure 13A: Influence of IkB on the DNA binding activity of various nuclear factors. The probes were: NF-kB; H2TF1, an oligonucleotide subcloned into pUC containing the H2TF1 binding site from the H-2 promoter; OCTA, an oligonucleotide subcloned into pUC containing the common binding site for the ubiquitous (upper filled arrowhead) and lymphoid-specific (lower filled arrowhead) octamer-binding proteins; NF-µE1; NF-kE2; and AP-1, EcoRI-HindIII fragment of the yeast HIS4 promoter containing three binding sites recognized by mammalian AP-1/jun. In the fluorograms shown, filled arrowheads indicate the positions of specific protein-DNA complexes. Open arrowheads indicate the positions of uncomplexed DNA fragments. Figure 13B: Interaction of IkB with NF-kB from different cell lines. The filled arrowheads indicate the positions of the NF-kB-DNA complexes from the various cell lines and the open arrowhead indicates the position of uncomplexed DNA probe.

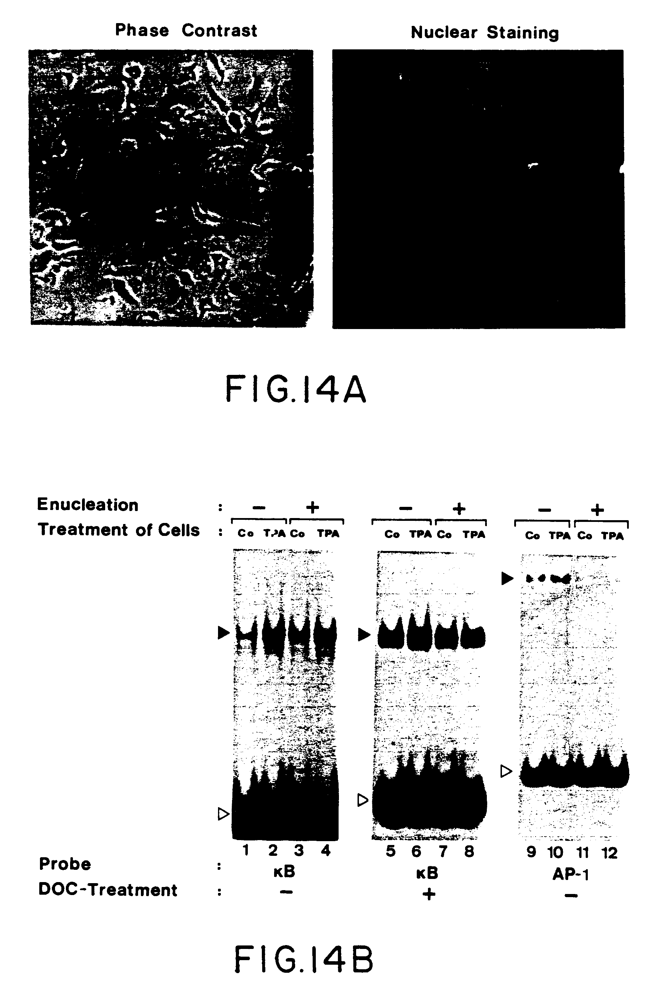

Figure 14 shows the presence of NF-kB in enucleated cells. Figure 14A: Phase contrast and fluoresence microscopy of enucleated HeLa cells. From 612 cells counted on photographic prints, 63 showed nuclear staining. A representative micrograph is shown. The arrow indicates a cell that retained its nucleus. Figure 14B: Analysis of complete and enucleated cells for NF-kB activity. Total cell extracts (1.2 µg of protein) from control (Co) and TPA-treated complete and enucleated cells were analyzed by EMSA with a labeled k enhancer fragment (kB) or HIS4 promoter fragment (AP-1) , 3 µg of poly(dI-dC), 1 µg of BSA, 1.2% NP-40® and the binding buffer in a final volume of 20 µl. In lanes 5 to 8, extracts were treated with DOC followed by the addition of the DNA binding mixture to give final concentrations of 0.8% DOC and 1.2% NP-40®. Samples were analyzed by EMSA. In the fluorograms shown, the filled arrowheads indicate the positions of specific protein-DNA complexes and the open arrowheads the positions of uncomplexed DNA probe.

### Detailed Description of the Invention

The present invention is based on the discovery that quantities of cytosolic NF-kB in fractions from unstimulated pre-B cells and of nuclear NF-kB from phorbol ester-activated cells are similar; that the NF-kB present in cytosolic fractions is inhibited in its DNA binding activity; and that the inhibition of cytosolic NF-kB can be removed by appropriate stimulation, which also causes translocation of NF-kB to the nucleus. It is also based on identification and localization of the precursor of NF-kB in the cytosolic fraction of unstimulated cells and on identification and characterization of a protein inhibitor, called IkB, which is present in the cytosol and can convert NF-kB into an inactive form in a reversible, saturable and specific reaction. The NF-kB precursor is present in a form in which DNA-binding activity can be activated in vitro by denaturing agents. The inhibitory activity of IkB is evident after selective removal of the endogenous cytosolic NF-kB under dissociating conditions, suggesting that NF-kB and IkB are present in a stoichiometric complex. The results described herein are consistent with a molecular mechanism of inducible gene expression by which a cytoplasmic transcription factor-inhibitor complex is dissociated by the action of TPA, presumably through activation of protein kinase C. The dissociation event has been shown to result in two events: activation (appearance of the NF-kB binding activity) and translocation of the factor to the nucleus.

Because NF-kB activity (and kappa gene transcription) can be induced, the state of NF-kB in unstimulated cells was assessed initially. Little NF-kB activity was demonstrable in either the nucleus or cytoplasm of unstimulated 70Z/3 cells. By a denaturation-renaturation protocol, NF-kB was shown to be present in cytosolic fractions, suggesting that the cytosolic NF-kB is bound to an inhibitor. Gentle dissociation of the inhibitor-NF-kB complex was effected, using dissociating agents; in particular, use of either sodium desoxycholate (DOC) or formamide revealed NF-kB in cytosolic fractions. Using equal cell-equivalents of protein and an optimized protocol, it was demonstrated that the quantity of cytosolic NF-kB in unstimulated 70Z/3 cells was as much as the quantity of nuclear NF-kB in phorbol ester-stimulated cells. HeLa cells have also been shown to contain inactive, cytosolic NF-kB. Subsequent assessment, as described below, of the DOC-dependence of cytosolic NF-kB demonstrated the presence of an activity in cytosolic fractions that specifically prevents DNA binding to NF-kB. The protein inhibitor, IkB, is present in cytosolic fractions of unstimulated pre-B cells, can convert NF-kB into an inactive DOC-dependent form by a reversible, saturable and specific reaction and appears to be present in a stoichiometric complex with NF-kB.

The following is a description of the occurrence and activation of NF-kB in cells which do not express k immunoglobulin light chain genes (and, in which NF-kB is not evident in either) cytoplasmic or nuclear fractions). In particular, the following is a description of localization of NF-kB in the cytosolic fraction; of activation of NF-kB in cytosolic fractions by dissociating agents; or redistribution of NF-kB into the nuclear fraction upon TPA stimulation; of demonstration of the appearance of NF-kB binding ability; and of the occurrence and characterization of an NF-kB inhibitor.

### NF-kB Occurrence and Activation in 70Z/3 cells

### NF-kB is Virtually Undetectable in Unstimulated 70Z/3 Cells

To determine where in the cell NF-kB or its inactive precursor are located, subcellular fractions from control and TPA-stimulated 7OZ/3 cells were analyzed for kB-specific DNA-binding activity. Nuclear extracts, cytosolic and postnuclear membrane fractions were analyzed at equal amounts of protein in an electrophoretic mobility shift assay, described in Example 1, followed by fluorography. (Sen, R. and D. Baltimore, Cell, 46:705-716 (1986) . The specificity of protein-binding to a fragment of the kappa light chain enhancer was controlled by using a fragment with a mutation in the binding motif for NF-kB. This mutation has been shown to prevent binding of NF-kB. Lenardo, M. et al., Science, 236:1573-1577 (1987) . Thus, any complexes formed on the wild type, but not on the mutant fragment, are considered specific for the NF-kB site.

Nuclear extracts from control cells contained very little kB-specific binding activity (Fig. 5, compare lanes 1 and 7) . This is in agreement with results reported previously by Sen and Baltimore. Sen, R. and D. Baltimore, cell, 46:705-716 (1986); Sen, R. and D. Baltimore, Cell, 47:921-928 (1986) . Similarly, the ctyosolic fraction produced only a faint, but specific and reproducible, signal co-migrating with the signal from the nuclear extract (Fig. 5, compare lanes 2 and 8) The fraction containing postnuclear membranes did not exhibit any detectable DNA-binding activity (Fig. 5, lane 3).

Upon treatment of cells with TPA for 30 minutes, the nuclear NF-kB activity was dramatically increased (Fig. 8, compare lanes 4 and 10). Almost no increase of the specific signal in the cytosolic fraction was observed (Fig. 5, compare lanes 5 and 11). The post-nuclear membrane fraction gave raise to an apparently kB-specific complex with a mobility higher than that formed by nuclear NF-kB (Fig. 5, compare lanes 6 and 12). None of the fractions had inhibitors of binding because added authentic NF-kB was fully recovered in all fractions, indicating that the results reflect a true activation of binding specificity.

### NF-kB is Detectable in the Cytosolic Fraction after Denaturation and Renaturation

To examine whether active NF-kB might be present but masked in fractions from unstimulated 70Z/3 cells, proteins from nuclear extracts and cytosolic fractions of control and TPA-stimulated cells were precipitated, denatured by boiling in SDS plus 2-mercaptoethanol and fractionated by electrophoresis through SDS-polyacrylamide gels. 300 ug of protein of nuclear extracts (N) and cytosolic fractions (C) from control (Co) and TPA-stimulated cells (TPA) were subjected to reducing SDS-polyacrylamide gel electrophoresis. Proteins eluted from different molecular weight fractions of the gel (i.e., corresponding to approximately 70-62 kDa (gel slice No. 6), 62-55 kDa (gel slice No. 7) and 55-48 kDa (gel slice No. 8)) were subjected to a renaturation protocol after removal of SDS. Hager, P.A. and R.R. Burgess, Anal. Biochem., 109:76-86 (1980) and Example 2. Renatured fractions were tested for kB - specific DNA-binding activity in mobility shift assays using wild type and mutant kappa light chain enhancer fragments. DNA-binding reactions were performed with 11 ul of the renatured fractions in the presence of 80 ng poly(d[I.c]) in a final volume of 15 ul. Assays with wild type (WT) and mutant (mu) k enhancer fragments were loaded in adjacent lanes.

In nuclear extracts from TPA-stimulated cells, NF-kB activity was exclusively found in a molecular weight region of 62-55 kDa. The efficiency of renaturation of the nuclear NF-kB activity was about one percent. In Figure 6, the active and two adjacent fractions are shown for the nuclear extract from TPA-stimulated cells (lanes 13 to 18) . In nuclear extracts from control cells, much less NF-kB activity was found in the same molecular weight fraction after renaturation (Fig. 6, lanes 3 and 4). Both the cytosolic fractions from control and TPA-stimulated cells, however, gave rise to a strong NF-kB-specific signal (Fig. 6, lanes 9, 10 and 21, 22). The specificity of the signal was shown by several criteria. First, it was only present when the wild type, but not the mutant, DNA fragment was used in mobility shift assays (Fig. 6, lanes 9, 10 and 21, 22) . Second, it was generated with protein eluted from the same molecular weight fraction that contained authentic nuclear NF-kB. Third, upon mixing, the complex formed by the putative cytoplasmic NF-kB co-migrated exactly in native polyacrylamide gels with the complex formed by interaction of the nuclear form of NF-kB with its cognate DNA.

Assuming that NF-kB from the various fractions had a similar recovery and efficiency of renaturation, the data suggest that significant amounts of NF-kB can be activated in unstimulated 70Z/3 cells by denaturation, followed by fractionation and renaturation. Furthermore, in unstimulated cells, the in vitro activated NF-kB activity was almost exclusively recovered in the cytosolic fraction.

The subcellular distribution of two noninducible DNA-binding proteins, NF-uE3 and the octamer-binding protein were also examined in mobility shift assays, in order to determine whether other DNA-binding factors also partition into cytoplasmic fractions. Sen, R. and D. Baltimore, Cell, 46:705-716 (1986); Singh, H. et al., Nature, 319:154-158 (1986); and Staudt, L.M. et al., Nature, 323:640-643 (1986). The vast majority of both DNA-binding activities was found in nuclear extracts; cytosolic and postnuclear membrane fractions contained only very little activities (Fig. 5, lanes 13 to 24). No significant change in the complex formation by the two factors was observed when fractions from control and TPA-stimulated cells were compared. Thus, although subcellular fractionation can produce artificial redistribution of proteins, the fractions used in this study do well reflect nuclear localization of a number of DNA-binding proteins.

### NF-kB in the Cytosolic Fraction Can be Activated by Dissociating Agents

The ability to reveal cytosolic NF-kB by simply denaturation and renaturation suggested that NF-kB might be bound to an inhibitor and therefore several compounds that might dissociate protein complexes were tested for their ability to directly activate kB-specific DNA-binding activity in fractions of 70Z/3 cells. The cytosolic fraction from unstimulated cells and, as a control, the nuclear extract from TPA- treated cells were incubated with the compounds prior to electrophoretic separation of the protein-DNA complexes. Incubation of the cytosolic fraction with 0.2% sodium desoxycholate (DOC) (in the presence of 0.2% NP-40®) resulted in activation of DNA-binding activity (Fig. 7A, lane 2). The induced complex had the same mobility in native gels as the one formed by nuclear NF-kB. It appeared to be specific for the kB site of the kappa light chain enhancer because it was not formed when the mutant fragment was used in the mobility shift assay (Fig. 7A, lane 21). Higher concentrations of DOC led to the inactivation of the newly activated kB-binding activity (Fig. 7A, lanes 3 to 5) as well as of the authentic nuclear factor (Fig. 7A, lanes 13 to 15).

DOC can be sequestered out of a solution by the addition of excess nonionic detergent, presumably by inclusion of the DOC into micelles formed by the nonionic detergent. When treatment of the cytosolic fraction with up to 0.8% DOC was followed by the addition of 1% of the nonionic detergent NP-40®, a quite efficient activation of the cytosolic kB-binding activity was achieved (Fig. 7A, lanes 7 to 9). The DNA-binding activity of in vivo activated NF- kB from nuclear extracts was not significantly increased at low concentrations of DOC (Fig. 7A, lanes 11, 12 and 16, 17) . Elevated concentrations of DOC showed inhibitory effects on the DNA-binding activity of TPA-activated NF-kB that paralleled those observed for the in vitro activated kB-binding activity (Fig. 7A, compare lanes 3, 4, 5 with lanes 13, 14, 15 and lanes 9, 10 with lanes 19, 20).

A partial activation of the cytosolic kB-binding activity was observed after treatment of the cytosolic fraction with 27% formamide followed by dilution (Fig. 7B, lane 7) . With the further addition of 0.2% DOC - - a condition that alone also leads only to partial activation (Fig. 7B, lane 3) - - a very potent activation was observed (Fig. 7B, lane 11). A titration showed that formamide and DOC activated in a synergistic manner (Fig. 7B, lanes 2 to 11). The DNA-binding activity of in vivo activated NF-kB from nuclear extracts was not enhanced by any of the treatments (Fig. 7B, lanes 13 to 22). On the contrary, partial inhibition of DNA-binding of NF-kB was observed under some conditions.

No in vitro activation of NF-kB was achieved by treatment with guanidinium hydrochloride (between 0.3 and 3M) , urea (between 0.5 and 5M) , and SDS (between 0.1 and 1%), in the presence of 0.2% NP-40®). Exhaustive dialysis of the cytosolic fraction using dialysis membranes with a cut-off of 25 kDa did not lead to an activation of DNA-binding activity. In the dialyzed fraction, NF-kB-activity could still be efficiently induced by formamide/DOC treatment, suggesting that no freely diffusible cofactors smaller than 25 kDa were required for the in vitro activation.

### TPA Stimulation Causes Redistribution of NF-kB into the Nuclear Fraction

To examine whether the form of NF-kB detected after in vitro activation in the cytosolic fraction could quantitatively account for the NF-kB found in nuclear extracts after TPA stimulation of cells, subcellular fractions of 70Z/3 cells were reinvestigated in mobility shift assays after treatment with formamide and DOC using equal cell-equivalents of subcellular fractions. Equal cell-equivalents of nuclear extracts (N) and cytosolic (C) and post-nuclear membrane fractions (P) from control (Co) and TPA-stimulated cells (TPA) were left untreated (lanes 1-6 and 13-18) or subjected to a formamide/desoxycholate treatment (Fa + DOC; lanes 7-12 and 19-24; for conditions see Fig. 7B, lab 11) . This treatment was preferred over the DOC/NP-40® chase treatment because it gave a higher resolution of bands in mobility shift assays. DNA-binding reactions were performed in the presence of 3.2 ug poly(d[I-C]) using 4.4 ug of protein from nuclear extracts, 8.8 ug of protein from cytosolic fractions or 2.2 ug of protein from postnuclear membrane fractions (all in 4 ul buffer D(+)). Fluorograms of native gels are shown. The specificity of protein-DNA complexes was controlled using wild type (kB wt) and mutant kappa enhancer fragments (kB mutant; see legend to Fig. 5) . The filled arrowhead indicates the position of kB-specific protein-DNA complexes and the open arrowhead the positions of unbound DNA-fragments.

Densitometric scanning of fluorograms showed that in control cells, more than 92% of the total cellular kB-specific DNA-binding activity was recovered in the cytosolic fraction following treatment with formamide and COD (Fig. 8, lanes 7 to 9). In TPA-stimulated cells. 80% of the kB-specific DNA-binding activity was found in nuclear extracts (Fig. 8, lanes 10 to 12). The remaining activity was largely recovered in the cytosolic fraction (Fig. 8, lane 11). All DNA-binding activities described were specific for the kB site, as shown by their absence when the mutant kappa enhancer fragment was used in the mobility shift assays (Fig. 8, lanes 13 to 24).

When the total cellular NF-kB activity that was activated in vitro in control cells was compared to the total cellular activity found in TPA-stimulated cells after the same treatment, virtually identical amounts of activity were observed. The equal amounts of NF-kB activity found in control and TPA-treated cells suggest that the treatment with formamide and DOC resulted in the complete conversion of an inactive precursor of NF-kB into a form of NF-kB with high DNA-binding affinity. Furthermore, these results provide evidence for a TPA-inducible translocation of NF-kB from the cytosol into the nucleus.

### NF-kB Occurrence and Activation in HeLa Cells

NF-kB activity can also be induced in HeLa cells after TPA treatment, as shown by the appearance of a kB-specific DNA-binding activity in nuclear extracts. Sen, R. and D. Baltimore, Cell, 47:921-928 (1986). Therefore, induction of NF-kB in the cytosolic fraction of HeLa cells was tested by treatment with formamide and DOC. To equal cell-equivalents of fractions, 17% formamide was added and diluted to 10% by the addition of the DNA-binding reaction mixture containing 4 ug poly(d[I-C]). DOC was then added to a final concentration of 0.6% to give a reaction volume of 20 ul. As says contained either 1.35 ug of protein from nuclear extracts, 9 ug of protein from the cytosolic fractions of 0.9 ug of protein from the postnuclear membrane fractions (all in 10 ul buffer D(+)).

Redistribution of NF-kB activity in the subcellular fractions upon TPA stimulation of cells, was also assessed, using the procedure described for 70Z/3 cells. Mobility shift assays were performed with equal cell-equivalents of the subcellular fractions. Because HeLa cells had about ten times as much cytosolic protein as nuclear protein -- as opposed to the 2:1 ratio in 70Z/3 cells - - the use of equal cell-equivalents of fractions gave very different quantitative results from those obtained with equal amounts of protein. Without any treatment, only traces of a kB-specific DNA-binding activity were detected in the nuclear and cytosolic fractions of HeLa cells and no activity was observed in the postnuclear membrane fraction (Fig. 9, lanes 1 to 3). Upon TPA stimulation of cells under the same conditions as for 70Z/3 cells, NF-kB activity was strongly increased in the nuclear extract (Fig. 9, lane 4) . Also, in the cytosolic fraction, a significant increase of NF-kB activity was found (Fig. 9, lane 5). This was not an artifact of fractionation because the activity of AP-1, another nuclear factor (Lee, W. et al., Cell, 49:741-752 (1987) , was highly enriched in nuclear extracts and almost not detectable in the cytosolic fraction of HeLa cells before and after TPA stimulation.

The treatment of control fractions of HeLa cells with formamide and DOC revealed large amounts of kB-specific DNA-binding activity in the cytosolic fraction (Fig. 9, compare lanes 8 and 20). The concentrations of formamide and DOC required for an optimal in vitro activation of NF-kB in HeLa cells were different from those required for 70Z/3 cells; less formamide and more DOG was needed. All DNA-binding activities described were specific for the kB-binding site in the kappa enhancer fragment (Fig. 9, lanes 13 to 24).

Almost no activity was detected in the HeLa nuclear extract and the postnuclear membrane fraction after in vitro activation (Fig. 9, lanes 7 and 9) . Large amounts of NF-kB activity could still be activated in the cytosolic fraction of TPA-stimulated HeLa cells (Fig. 9, lane 11). This suggests that in vivo in HeLa cells - - as contrasted to 70Z/3 cells - - only a minor portion of the total cellular NF-kB is activated upon a TPA stimulus. The NF-kB activity in formamide/DOC-treated nuclear extracts of TPA-stimulated cells was less, compared to untreated nuclear extracts (Fig. 9, compare lanes 4 and 10), reflecting a partial inhibition of the DNA-binding activity of in vivo activated NF-kB. As in 70Z/3 cells, the total cellular NF-kB activity in HeLa cells, as revealed after in vitro activation, remained constant before and after TPA treatment of cells. These data imply that NF-kB is activated by the same mechanism in HeLa cells as it is in the pre-B cell line 70Z/3. However, in HeLa cells, TPA is much less complete in its activation than it is in 70Z/3 cells.

### NF-kB Occurrence and Activation in Other Cell Types

NF-kB occurrence and activation in several additional cell types, including two T cell lines (H9, Jurkat) and fibroblasts, and in tissues, including human placenta and mice kidney, liver, spleen, lung, muscle and brain, were also assessed, as described above. In each case, NF-kB in a DOC-activatable form was shown to be present in the cytosolic fraction.

### Appearance of Binding Activity

Results described above suggested that the appearance of binding activity may be due to separation of NF-kB from an inhibitor. Size fractionation and denaturing agents were both shown to be capable of separating NF-kB from such an inhibitor, which was apparently of low molecular weight. This provides a reasonable explanation for how NF-kB is induced in pre-B cells, HeLa cells and other inducible cells, such as T cells.

Whether the DOC-dependence of cytosolic NF-kB results from its association with an inhibitor, was investigated by probing for activity in cytosolic fractions that would specifically prevent DNA binding to NF-kB in electrophoretic mobility shift assays (EMSA). This work demonstrated the existence of a protein inhibitor, called IkB, in cytosolic fractions of unstimulated pre-B cells, that can convert NF-kB into an inactive DOC-dependent form by a reversible, saturable, and specific reaction. The inhibitory activity becomes evident after selective removal of the endogenous cytosolic NF-kB under dissociating conditions, suggesting that NF-kB and IkB were present in a stoichiometric complex. Enucleation experiments showed that the complex of NF-kB and IkB is truly cytoplasmic. The data are consistent with a molecular mechanism of inducible gene expression by which a cytoplasmic transcription factor-inhibitor complex is dissociated by the action of TPA, presumably through activation of protein kinase C. The dissociation event results in activation and apparent nuclear translocation of the transcription factor. It would appear that IkB is the target for the TPA-induced dissociation reaction. The following is a description of this investigation, which is described in greater detail in Example 4.

### Separation of an Inhibitor from NF-kB

Cytosolic fractions from unstimulated 70Z/3 pre-B cells were examined for an activity that would impair the DNA binding activity of added NF-kB in an EMSA. Baeuerle, P.A. , and D. Baltimore, Cell 53: 211 (1988). Increasing amounts of cytosol from unstimulated cells did not significantly influence the formation of a protein-DNA complex between NF-kB and a k enhancer fragment (Figure 10, lanes 13 to 15). This indicated the absence of free inhibitor, presumably because all of it is complexed with endogenous NF-kB. DNA-cellulose was used to selectively remove the endogenous NF-kB from DOC-treated cytosol, in an attempt to liberate the inhibitor. Almost all NF-kB was present in a DOC-dependent form prior to DOC activation and chromatography (Figure 10A, lanes 1 and 2) . In the presence of excess DOC, about 80% of the NF-kB activity was retained by DNA-cellulose (Figure 10A, compare lanes 2 and 4) , most of which eluted from the DNA-cellulose between 0.15 and 0.35M NaCl (Figure 10A, lanes 8 to 10 and 16 to 18) . The NF-kB activity eluting at high salt was detectable in mobility shift as says in the absence of excess DOC (Figure 10A, lanes 8 to 11), indicating that NF-kB had been separated from an activity that caused its DOC-dependent DNA binding activity. In contrast, the small percentage of NF-kB activity contained in the washings was still dependent on DOC (Figure 10A, compare lanes 5 to 7 and 13 to 15). These results show that affinity chromatography is sufficient to convert DOG-dependent NF-kB precursor into DOC-independent active NF-kB, similar to that found in nuclear extracts from TPA-stimulated cells.

The flow-through fraction from the DNA-cellulose was assayed for an activity that, after neutralization of DOC by non-ionic detergent, would inactivate added NF-kB from the 0.2M NaCl-fraction from nuclear extracts of TPA-stimulated cells. Increasing amounts of cytosol from which the endogenous NF-kB was removed inhibited the formation of an NF-kB--DNA complex as monitored by EMSA (Figure 10B, lanes 7 to 9 and 16 to 18). DOC-treated cytosol that was not passed over DNA-cellulose had no effect (Figure 10B, lanes 4 to 6 and 13 to 15), even if cells had been treated with TPA. The fact that, after DNA-cellulose chromatography of DOC-treated cytosol, both DOC-independent NF-kB and an inhibitory activity were observed made it reasonable to believe that NF-kB had been separated from an inhibitor. This inhibitor is referred to as IkB.

### IkB Characterization

IkB fractionates as a 60 to 70 kD protein. The flow-through fraction from the DNA-cellulose column was subjected to gel filtration through G-200 Sephadex® and the fractions were assayed for an activity that would interfere with the DNA binding activity of added NF-kB contained in a nuclear extract from TPA-stimulated 70Z/3 cells (Figure 11A). The 67 kD fraction had the highest activity: it virtually completely prevented interaction of NF-kB and DNA (Figure 11A, lanes 6). In fractions from a G-75 Sephadex® column, no additional inhibitor of low molecular size was detectable indicating that NF-kB was inactivated by a macromolecule of defined size. No significant inhibitory activity could be demonstrated after gel filtration of a DNA-cellulose flow-through of DOC-treated cytosol from TPA-stimulated 70Z/3 cells, implying that TPA treatment of cells inactivated IkB. Baeuerle, P.A. and D. Baltimore, unpublished observation.

The inhibitor fraction was treated with trypsin to test whether IkB is a protein (Figure 11B) . Tryptic digestion was stopped by the addition of bovine pancreas trypsin inhibitor (BPTI) and samples were analyzed for NF-kB inhibition. Trypsin treatment interfered with the activity of IkB, as shown by the complete inability of the treated sample to inhibit NF-kB activity (Figure 11B, compare lanes 1 and 6). Trypsin that had been treated with BPTI had no effect (Figure 11B, lane 5), demonstrating that the inactivation of IkB was specifically caused by the proteolytic activity of trypsin. It appears that IkB requires an intact polypeptide structure for its activity.

The cytosolic complex of IkB and NF-kB showed an apparent size of about 120 to 130 kD, both after gel filtration (Figure 11A, lane 3) and after sedimentation through a glycerol gradient (Figure 11C, lanes 6 and 7) For both methods, cytosol from unstimulated cells was analyzed under non-dissociating conditions. NF-kB was activated in fractions by either DOG (Figure 11A) or formamide (Figure 11C, middle panel) prior to analysis by EMSA. Baeuerle, P.A. and D. Baltimore, Cell, 53:211 (1988). The specificity of complexes was tested with a mutant DNA probe (Figure 11C, right panels). Lenardo, M. et al. , Science, 236:1573 (1987). The apparent release of a 60 to 70 kD inhibitory protein from the cytosolic NF-kB precursor, its sedimentation velocity in glycerol gradients, and its size seen by gel filtration suggest that the inactive NF-kB precursor is a heterodimer composed of a 55 to 62 kD NF-kB molecule and a 60 to 70 kD IkB molecule. Nuclear NF-kB was found to cosediment with the cytosolic complex of IkB and NF-kB (Figure 11C, upper panel). Native gel electrophoresis, a method that allows resolution of size differences of protein-DNA complexes, provided evidence that the 120 kD form of nuclear NF-kB seen in glycerol gradients comes from the formation of a homodimer. Hope, I.A. and K. Struhl, EMBO J ., 6:2781 (1987) and Baeuerle, P.A. et al., unpublished observation. By these interpretations, activation of NF-kB would include an additional step (i.e. , formation of a NF-kB homodimer). This is consistent with the observation that the protein-DNA complexes formed with in vitro-activated NF-kB have the same mobility in native gels as those formed with nuclear NF-kB. Baeuerle, P.A. and D. Baltimore, Cell, 53:211 (1988).

The inactivation of NF-kB by IkB is reversible, saturable and specific. Incubation with the inhibitor fraction can inhibit the DNA binding activity of NF-kB by more than 90% (Figure 12A, lanes 1 and 3) . Treatment of a duplicate sample with DOG after the inhibition reaction reactivated 66% of the added NF-kB activity (Figure 12A; compare lanes 3, 4 and 6) . This showed that a DOC-dependent form of NF-kB can be reconstituted in vitro by the addition of a fraction containing IkB to nuclear NF-kB. The incomplete activation of NF-kB by DOC might be due to the DOC-neutralizing effect of non-ionic detergent which was still present in the sample from the preceding inhibition reaction.

A titration and kinetic analysis showed that IkB stoichiometrically interacts with NF-kB (Figure 12B). Increasing amounts of inhibitor fraction were added to an excess amount of NF-kB and incubated for 20 or 60 minutes. After the DNA binding reaction, NF-kB-DNA complexes were separated on native gels and quantified by liquid scintillation counting. The relationship between amount of IkB fraction added and extent of inhibition was linear. The amount of NF-kB inactivated after 20 minutes of incubation was not increased after 60 minutes (Figure 12B). These kinetics were probably not the result of a rapid decay of a catalytically active inhibitor because the fractions were incubated prior to the reaction. The data are consistent with rapid formation of an inactive complex by addition of IkB to NF-kB. The fraction containing IkB does not appear to catalytically or covalently inactivate NF-kB: neither the reversibility nor the kinetics support such a model.

IkB was tested for its influence on the DNA binding activity of other defined nuclear factors (Figure 13A). These factors were contained in nuclear extracts that had essentially no active NF-kB, which otherwise could have inactivated IkB by complex formation. The DNA binding activity of H2TF1, a transcription factor thought to be related to NF-kB, was not affected by the inhibitor fraction. Baeuerle, P.A. et al. , unpublished observation). Ubiquituous and lymphoid-specific octamer-binding proteins (OCTA) (Sive, H.L. and R.G. Roeder, Proc. Natl. Acad. Sci. USA, 83:6382 (1986) and Staudt, L.M. et al., Nature, 323:640 (1986)) were unaffected in their DNA binding activities, as were two E-box binding factor, NF-µE1 (Weinberger, J. et al., Nature, 322:846 (1986)) and NF-kE2 (Lenardo, M. et al., Science, 236:1573 (1987)), interacting with µ heavy chain and k light chain enhancers, respectively. AP-1, another TPA-inducible transcription factor (Lee, W. et al. , Cell, 49:741 (1987); Angel, P. et al. , Cell, 49:729 (1987)), also showed equal complex formation after incubation in the presence and absence of the inhibitor fraction. Furthermore, none of the undefined DNA binding activities seen in the EMSA showed any inactivation by IkB. These results show that IkB is a specific inhibitor of the DNA binding activity of NF-kB.

In vivo activated NF-κB is responsive to IkB. IkB prepared from the mouse pre-B cell line 70Z/3 was tested for inactivation of NF-kB contained in nuclear extracts from other cell lines. Human NF-kB contained in nuclear extracts from TPA-stimulated HeLa cells and H-9 T-lymphoma cells was efficiently inactivated (Figure 13B). When excess amounts of the various NF-kB activities were used in the inhibitor assay, the extent of reduction of NF-kB activities by a fixed amount of IkB was very similar, as quantified by liquid scintillation counting. Baeuerle, P.A. and D. Baltimore, unpublished observation. NF-kB from nuclear extracts of TPA-stimulated Madin-Darby bovine kidney (MDBK) cells was also inactivated suggesting that the control of NF-kB activity by IkB is conserved among different mammalian species. Baeuerle, P.A. and D. Baltimore, unpublished observation.

NF-kB is constitutively active in cell lines derived from mature B cells. Sen, R. and D. Baltimore, Cell, 46:705 (1986). Nuclear extracts from the mouse B cell line WEHI 231 were tested in the inhibitor assay to examine whether NF-kB has undergone a modification in those cell lines that prevented its inactivation by IkB. NF-kB from B cells was as efficiently inactivated as NF-kB from pre-B cells (Figure 13B), suggesting that NF-kB is not stably modified in B cells (or in other cells after TPA stimulation) in such a way that it cannot respond to inactivation by IkB.

The NF-kB--IkB complex is present in enucleated cells. The NF-kB--IkB complex shows a cytosolic localisation on subcellular fractionation (Figure 14A). This procedure may, however, cause artifacts. Hypotonic lysis of cells may result in partitioning of nuclear proteins into the cytosol, especially, when they are not tightly associated with nuclear components. Li, J.J. and T.J. Kelly, Proc. Natl. Acad. Sci, USA, 81:6973 (1984). Detection of the complex of IkB and NF-kB in enucleated cells was attempted. Enucleation is performed with living cells at 37°C and should therefore not interfere with active nuclear import of proteins, which is ATP-dependent and blocked at low temperature. Prescott, D.M. and J.B. Kirkpatrick, In: Methods Cell Biol., D.M. Prescott, ed. (Academic Press, New York, 1973), p. 189; Newmeyer, D.P. and D.J. Forbes, Cell, 52:641 (1987); Richardson, W.D. et al., Cell, 52:655 (1988).

Using cytochalasin B-treated HeLa cells, an enucleation efficiency of about 90% was obtained (Figure 14A). Enucleated and cytochalasin B- treated complete cells were incubated in the absence and presence of TPA, solubilized by detergent and proteins were extracted with high salt. Because of the small number of cells analyzed, this procedure is different from the standard one. Total cell extracts were analyzed for NF-kB specific DNA binding activity by EMSA (Figure 14B). In both enucleated and complete cells, similar amounts of NF-kB activity were found after TPA stimulation (Figure 14B, lanes 1 to 4). The activity was specific for NF-kB because it was not observed with a mutant k enhancer fragment. Lenardo, M. et al., Science, 236:1573 (1987); Baeuerle, P.A. and P. Baltimore, unpublished observation. These results suggest that TPA-inducible NF-kB in HeLa cells is predominantly cytoplasmic because it was still present in enucleated cells. The NF-kB activity seen under control conditions (Figure 14B, lanes 1 and 3) was most likely activated by the lysis conditions used because it was also observed in extracts from HeLa cells that were not treated with cytochalasin B (Baeuerle, P.A. and D. Baltimore, unpublished observation) , but not in fractions obtained after hypotonic lysis. Baeuerle, P.A. and D. Baltimore, Cell, 53:211 (1988). It was still evident, however, that TPA could activate NF-kB in enucleated sells (Figure 14B, lanes 3 and 4).

After treatment with DOC, total extracts from complete and enucleated control cells showed about a 2-fold increase in the amount of NF-kB activity (Figure 14B, compare lanes 1 and 3 with 5 and 7). The demonstration of DOC-activatable NF-kB in enucleated cells, as well as the presence of similar amounts of total NF-kB in enucleated and complete cells (Figure 14B, compare lanes 5 to 8) , shows that a substantial amount of the total cellular NF-kB--IkB complex was cytoplasmic.
In contrast to NF-kB, most of the DNA binding activity of AP-1, a bona fide nuclear protein, was apparently lost by enucleation of cells (Figure 14B, lanes 9 to 12) . Lee, W. et al., Cell, 49:741 (1987); Angel, P. et al., Cell, 49:729 (1987).

### Mechanism of NF-kB activation

Thus, it has been shown that the NF-kB nuclear transcription factor exists in unstimulated pre-B cells in a cytoplasmic complex with a specific inhibitory protein, IκB. In this complex, NF-kB does not exhibit DNA binding activity in EMSA and partitions upon subcellular fractionation into the cytosol. The complex is apparently a heterodimer consisting of about a 60 kD NF-kB molecule and a 60 to 70 kD IkB molecule. Upon TPA stimulation of cells, or after treatment with dissociating agents in vitro, the NF-kB--IkB complex dissociates. This releases NF-kB, which appears now to form a homodimer and can translocate into the nucleus. Whether dimerization is required for activation of NF-kB is not known.

The inhibitory effect of IkB on the DNA binding activity and nuclear localization properties of NF-kB appears to arise from a simple physical affinity of the two proteins. The complex freely dissociates and the components readily associate under in vitro conditions. Even in vivo, dissociation by short-term TPA treatment and reassociation after long-term TPA treatment is evident. Baeuerle, P.A. et al., Cold Spring Harbor Symp. Quant. Biol., 53, In Press. The latter presumably results from the degradation of protein kinase C after TPA activation and implies that NF-kB can move back to the cytoplasm after being active in the nucleus.

The effect of TPA appears to involve an alteration of IkB, but not of NF-kB. After TPA stimulation, no active IkB was found - - implying its alteration - - while the nuclear NF-κB remained sensitive to unmodified IkB when tested in vitro. Whether inactive IkB can be regenerated is unclear; in experiments using cycloheximide (Baeuerle, P.A. et al., Cold Spring Harbor symp. Quant. Biol., 53, In Press), irreversible loss of IkB activity was the only demonstrable effect after 8 hours of TPA treatment. Given the ability of TPA to activate protein kinase C, it is a reasonable hypothesis that direct or indirect phosphorylation of IkB results in its dissociation from NF-kB.

It had previously been found that the NF-kB--IkB complex is recovered in the cytosol. It is now shown directly that the complex is not removed from the cell by enucleation and, therefore, is truly cytoplasmic. Welshons, W.V. et al., Nature, 307:747 (1984). Because active protein kinase C is bound to the plasma membrane (Kraft, A.S. et al., J. Biol. Chem., 257:13193 (1983); Wolf, M. et al., Nature, 317:546 (1985); Kikkawa, U. and Y. Nishisuka, Ann. Rev. Cell. Biol., 2:149 (1986)), it becomes increasingly attractive to suggest that the cytoplasmic complex interacts in the cytoplasm (maybe near the plasma membrane) with protein kinase C and the liberated NF-kB carries the signal from cytoplasm to nucleus. Under a number of conditions, active NF-kB is found in the cytoplasm. This fact and the reversibility of NF-kB activation in vivo suggests that the protein may freely move in and out of the nucleus, bringing to the nucleus information reflecting the cytoplasmic activation state of protein kinase C and possibly of other signalling systems.

The response of NF-kB to activated protein kinase C occurs apparently indirectly through modification and subsequent release of associated IkB. The inducibility of NF-kB by TPA is thus dependent on the presence and state of activity of IkB. Changes in amount or activity of IkB should therefore influence the TPA inducibility of NF-kB. NF-kB can indeed exist not only in TPA-inducible but also in constitutively active form (e.g., in mature B cells; Sen, R. and D. Baltimore, Cell, 46:705 (1986). Because constitutive NF-kB from B cells is still responsive to IkB in vitro, it is thought that IkB, and not NF-kB, is altered during differentiation of pre-B into B cells.

IkB is apparently unstable when not complexed with NF-kB. This is suggested by the absence of excess active inhibitor in the cytosol from unstimulated cells. In a situation where the production of new inhibitor is impaired, the decay of occasionally released inhibitor could activate NF-kB. This would explain the partial activation of NF-kB seen after treatment with the protein synthesis inhibitors cycloheximide and anisomycin. Sen R. and D. Baltimore, Cell, 47:921 (1987). The demonstration of a specific inhibitory protein of NF-kB and the interpretation that cycloheximide treatment can activate NF-kB, presumably because cells become depleted of inhibitor, suggest that IkB is the putative labile repressor of k gene expression (Wall, R. et al., Proc. Natl. Acad. Sci. USA, 83:295 (1986)) and of NF-kB activity. Sen, R. and D. Baltimore, Cell, 47:921 (1987).

### Role of NF-kB in HIV Expression

Treatment of latently HIV-infected T-cells with phorbol ester (12-O-tetradecanoylphorbol 13-acetate: TPA) and with phytohaemaglutinin (PHA) results in the onset of virus production. Harada, S. et al., Virology, 154:249-258 (1986); Zagury, D.J. et al., Science, 232:755-759 (1986). The same treatments induce NF-kB activity in the human T-lymphoma cell line Jurkat. Sen, R. and D Baltimore, Cell, 47:921-928 (1896). This correlation and the finding that two NF-kB binding sites are present upstream of the transcriptional start site in the HIV enhancer, (Figure 1) suggested a direct role for NF-kB in the activation of the viral enhancer, an event ultimately leading to the production of virus. Nabel, G. and D. Baltimore, Nature, 326:711-713 (1987). This possibility was tested by transient transfection of a plasmid containing an HIV LTR-controlled CAT gene into a human T-lymphoma cell line. Nabel, G. and D Baltimore. Nature, 326:711-713 (1987). The viral cis-acting elements rendered the transcriptional activity of the CAT gene responsive to TPA/PHA treatment of cells. This inducible transcriptional stimulation of the CAT gene was completely dependent on intact binding sites for NF-kB in the HIV enhancer because mutation of the two binding sites abolished inducibility. A protein-DNA complex with a fragment of the HIV enhancer containing the two NF-kB binding sites was observed in mobility shift assays only with nuclear extracts from TPA/PHA-stimulated T-cells and not with control extracts. These observations provided strong evidence that HIV express ion in latently infected T - cells is induced by the same transcription factor that regulates kappa gene expression, NF-kB. A precursor of NF-kB is constitutively present in T-cells. Its activity can be induced by a treatment that mimicks antigenic T-cell activation and, after induction, NF-kB is able to bind to and subsequently enhance the activity of HIV transcriptional control elements. Thus, it is reasonable to conclude that NF-kB is the physiological trans-activator responsible for initial expression of dormant HIV-DNA following stimulation of T-lymphocytes.

Other factors have also been implicated in the control of HIV expression including the HIV-encoded tat-III protein, the cellular transcription factor Spl, and viral proteins encoded by the E1A gene of adenovirus and the ICPO gene of the Herpes Simplex Virus. Muesing, M.A. et al., Cell, 48:691-701 (1987); Jones, K.A. et al., Science, 232:755-759 (1986); Gendelman, H.E. et al., Proceedings of the National Academy of Sciences, USA, 83:9759-9763 (1986); Nabel, G.J. et al., Science (1988); Rando, R.F. et al., Oncogene, 1:13-19 (1987); Mosca, J.D. et al., Nature, 325:67-70 (1987). It is doubtful whether the tat-III and Spl proteins are responsible for an initial induction of HIV expression. Although the tat-III protein functions as a strong positive feedback regulator of HIV expression, full expression of the tat-III protein appears to depend on NF-kB. Muesing, M.A. et al., Cell, 48:691-701 (1987); Nabel, G. and D. Baltimore, Nature, 326:711-713 (1987). It is unlikely that Spl initiates HIV expression because it is constitutively active. Dynan, W.S. and R. Tjian, Cell, 32:669-680 (1983). The viral E1A and ICPO gene products might lead to induction of HIV expression. This, however, is independent of T-cell activation by antigenic stimulation and of NF- kB, as shown by cotransfection experiments into human T-lymphoma cells of plasmids with an HIV enhancer-controlled CAT gene and plasmids encoding the viral genes. The increase in CAT activity induced by the viral gene products was unchanged when the NF-kB binding sites in the HIV enhancer were inactivated by mutation.

### Characterization of the NF-kB Protein

Mouse NF-kB is a polypeptide with a molecular weight around 60 kDa. This has been determined by a DNA-binding renaturation experiment using eluates from different molecular weight fractions of a reducing SDS-gel (Figure 2A). The size of the native NF-kB protein was determined in the following manner: Nuclear extract from TPA-stimulated cells was subjected to ultracentrifugation on a continuous glycerol gradient. The fractions were assayed for DNA-binding activity of NF-kB by electrophoretic mobility shift assays (Figure 2B). NF-kB activity was found highest between the co-sedimented bovine serum albumin (67 kDa) and IgG (158 kDa) standards (Figure 2B, lanes 6 to 8). The specificity of binding was shown by the absence of a complex when a DNA probe with a mutation in the NF-kB binding sequence was used to assay the fractions (Figure 2B, right lanes 4 to 10). Lenardo, M. et al., Science, 236:1573-1577 (1987). Little NF-kB activity was contained in the fractions where a 60 kDa protein would be expected to sediment (Figure 2B, lanes 4 and 5). If the sedimentation of NF-kB is not highly abnormal, the results from the glycerol gradient centrifugation suggest that NF-kB is associated with another protein of approximately the same size. Presumably NF-kB forms a homodimer because the protein-DNA complex formed in native gels using whole nuclear extract is of the same mobility as the complex formed with renatured NF-kB protein from a single spot of a two-dimensional gel.

The present invention is useful as a means of controlling activation in a host cell of an NF-kB precursor, which results in formation of activated NF-kB, which, in turn, plays a key role in transcriptional activation of other sequences, such as the k light chain enhancer, the HIV enhancer and the interleukin-2 receptor a-chain gene. NF-kB has been shown to be a ubiquitous inducible transcription factor; it has been shown, as described herein, to be present in many types of cells (i.e., all cell types assessed to date) . It serves to make immediate early responses which it is capable of effecting because it is post-translationally activated. As a result, the method and composition of the present invention can be used to control transcriptional activation of genes encoding a selected cellular protein. Changes in expression of genes transcribed by RNA polymerase II in response to agents, such as steroid hormones, growth factors, interferon, tumor promoters, heavy metal ions and heat shock are mediated through cis-acting DNA sequence elements such as enhancers. Binding of NF-kB transcription factor has been shown to confer transcriptional activity on several genes. Expression of these genes and others similarly affected can be controlled by the present method. For example, it has been shown that expression of one of the two elements of the cell surface receptor specific for IL-2 is controlled by NF-kB. Thus, in T cells, which produce IL-2, production can be controlled (enhanced, reduced) by controlling activation of NF-kB. In a similar manner, the method of the present invention can be used to control expression of human immunodeficiency virus in infected host cells.

The present invention will now be illustrated by the following examples, which are not to be seen as limiting in any way.

### EXAMPLE 1 Electrophoretic Mobility Shift Analysis of Subcellular Fractions of 70z/3 Cells

70Z/3 cell cultures were incubated in the absence (Co) and presence of phorbol ester (TPA), followed by subcellular fractionation of cells. In the DNA-binding reactions, 8.8 ug of protein of nuclear extracts (N), cytosolic fractions (C), and post-nuclear membrane fractions (P) in 4 ul buffer D(+) were used. The end-labeled DNA-fragments were incubated in the presence of 3.2 ug poly(d[I-C]) with the subcellular fractions in a final volume of 20 ul for 15 to 30 minutes followed by separation of protein-DNA complexes and unbound DNA on native 4% polyacrylamide gels. Fluorograms of native gels are shown. To detect kB-specific DNA-binding activity a DdeI-HaeIII wild type fragment of the kappa light chain enhancer (kB wt; lanes 1-6) was used. Sen, R. and D. Baltimore, Cell, 46:705-716 (1986). kB-unspecific activities binding to the kappa enhancer fragment were detected using a fragment mutated in NF-kB binding site that was otherwise identical to the wild type fragment (lanes 7-12). Lenardo, M. et al., Science, 236:1573-1577 (1987). NF-uE3-binding activity and octamer binding protein activity were assayed with a HaeIII-DdeI kappa enhancer fragment (uE3; lanes 13-18) and a PvuII-EcoRI kappa heavy chain promoter fragment (OCTA; lanes 19-24), respectively. Sen, R. and D. Baltimore, Cell, 46:705-716 (1986); Singh, H. et al., Nature, 319:154-158 (1986). Specific protein-DNA complexes are indicated by filled arrowheads and the positions of unbound DNA-fragments by open arrowheads.

### EXAMPLE 2 Renaturation of NF-kB

70Z/3 cells were grown in spinner cultures with RPMI 1640 medium supplemented with 10% newborn calf serum and 50 uM 2-mercaptoethanol. HeLa cells were also grown in spinner cultures with MEM medium supplemented with 10% horse serum. Cell cultures were treated with 25 ng/ml 12-O-tetradecanoylphorbol 13-acetate (TPA; Sigma) for 30 minutes at cell densities between 7x10⁵ and 2x10⁶/ml.

### Subcellular Fractionation

Cells were collected by centrifugation for 1o minutes at 150xg. Cell pellets were resuspended in ice-cold phosphate-buffered saline and collected again by centrifugation. All following steps were carried out at 4°C. Washed cells were resuspended in four packed cell volumes of a hypotonic lysis buffer (buffer A; Dignam, J. P. et al., Nucleic Acid Research, 11:1475-1489 (1983)). After 20 minutes, cells were homogenized by 15 (HeLa) or 20 strokes (70Z/3 cells) with a loose fitting Dounce homogenizer. Nuclei were collected by centrifugation for 6 minutes at 4300xg, resuspended in five volumes of buffer A and washed once by centrifugation. Proteins were extracted from washed nuclei by high salt, followed by centrifugation of the nuclear extracts and dialysis against buffer D as described. Dignam, J.P. et al., Nucleic Acids Research, 11:1475-1489 (1983). One percent NP-40 (v/v) was added to the dialyzed nuclear extracts. The postnuclear supernatant was centrifuged for 6 minutes at 4300xg and the resulting supernatant ultracentrifuged for 1 hour at 150,000xg. The pellet after ultracentrifugation containing postnuclear membranes was dissolved in buffer D containing 1% (v/v) NP-40 (referred to as buffer D(+)). Insoluble material was removed by centrifugation for 10 minutes in a Microfuge. The supernatant after ultracentrifugation (referred to as cytosolic fraction) was adjusted to buffer D(+) conditions by the addition of stock solutions. Fractions were stored at -70°C.

Protein concentrations were determined by an assay using bicinchoninic acid. Smith, P.K. et al., Anals of Biochemistry, 150:76-85 (1985). The ratio of the total protein recovered during a fractionation experiment in nuclear extracts, cytosolic fractions and postnuclear membrane fractions was 2:4:1 for 70Z/3 cells and 1:10:1.5 for HeLa cells. These ratios were used to adjust the fractions to protein concentrations reflecting equal cell-equivalents of subcellular fractions.

### Electrophoretic Mobility Shift Assays and Treatments with Dissociating Agents

DNA-binding reactions were carried out as described above. The DNA-binding reaction mixture contained poly(d[I-C]) (Pharmacia), 3000-6000 cpm of [³²P]end-labeled DNA-fragments and a buffer composed of 10 mM Tris-HCl, pH 7.5, 50 mM NaCl, 1 mM dithiothreitol (DTT) , 1 mM EDTA and 5% glycerol. Binding reactions and subsequent analysis on native 4% polyacrylamide gels were performed at room temperature as described. Sen, R. and D. Baltimore, Cell, 46:705-716 (1986). Subcellular fractions were treated with formamide (DNA grade; American Bioanalytical) prior to the addition of the DNA-binding reaction mixture. Sodium desoxycholate (Fisher Scientific Company) was added after the DNA-binding reaction.

### Renaturation of NF-kB

Protein in the subcellular fractions was precipitated at -20°C by the addition of four volumes of acetone. Pellets were dissolved in SDS-sample buffer containing 3.3% 2-mercaptoethanol and boiled for 5 minutes. Laemmli, U.K. , Nature, 227:680-685 (1970). After SDS-polyacrylamide gel electrophoresis, gel pieces from different molecular weight regions were cut out, ground, and proteins eluted overnight at 4°C in 500 ul of a buffer containing 50 mM Tris-HCl, pH 7.9, 0.1% SDS, 0.1 mg/ml bovine serum albumin, 1 mM OTT, 0.2 mM EDTA, 0.1 mM phenylmethylsulfonyl fluoride (PMSF) and 2.5% glycerol. After centrifugation for 2 minutes in a Microfuge, the supernatant was removed and recentrifuged for 10 minutes to remove gel debris.

To 200 ul of the supernatant four volumes of acetone were added and proteins were allowed to precipitate for 2 hours at -20°C. The precipitate was collected by centrifugation for 10 minutes in a Microfuge, washed once with 1 ml methanol at -20°C and dried for 30 minutes in the inverted tube. The dried pellet was dissolved in 2.5 ul of a saturated solution of urea (ultrapure; American Bioanalytical) and dilued with 125 ul of a buffer containing 20 mM Tris-HCl, pH 7.6, 10 mM KCl, 2 mM DTT and 10 uM PMSF. Renaturation was allowed for a minimum of 18 hours at 4°C. kB-specific DNA-binding activity was detectable in mobility shift assays for at least 48 hours after storage of renatured fractions at 4°C without appreciable loss of activity.

### EXAMPLE 3 Subcellular Localization of the NF-kB Precursor

Because NF-kB is a DNA-binding protein, it is expected to reside in the nucleus. This is certainly true for NF-kB of phorbol ester-treated cells and mature B-cells where the activity is detectable in nuclear extracts. It is however not mandatory for a precursor of NF-kB especially, in view of the fact that the precursor is activated by protein kinase C, a cytosolic protein that is associated in its active state with the plasma membrane.

The precursor of NF-kB was analyzed by an investigation of subcellular fractions of unstimulated pre-B cells for the presence of NF-kB activity, using electrophoretic mobility shift assays (Figure 3A). Little DNA-binding activity was detected in the subcellular fractions, indicating that the precursor must exist in a form of low affinity for its cognate DNA (Figure 3A, lanes 1 to 3). In fractions from TPA-stimulated cells, the newly activated NF-kB was almost exclusively contained in the nuclear extract (Figure 3A, lanes 4 to 6).

In an attempt to activate the DNA-binding activity of the NF-kB precursor, the various subcellular fractions were treated with agents known to gently dissociate protein-protein interactions. Low concentrations of desoxycholate, formamide or a combination of both included in the mobility shift assay mixture led to the activation of an NF-kB-specific DNA-binding activity (Figure 3B). The fraction containing the bulk of the in vitro activatable NF-kB precursor was the cytosol (Figure 3B, lanes 1 to 3). When fractions from TPA-stimulated cells were subjected to the same treatment, the amount of precursor in the cytosolic fraction was found strongly reduced, apparently because of redistribution of activated NF-kB into the nuclear extract fraction (Figure 3B, lanes 4 and 5). In both control and TPA-stimulated cells, the amount of total cellular NF-kB activity revealed after treatment with dissociating agents was equal, suggesting a complete conversion of the NF-kB precursor into active NF-kB. Cytosolic fractions from HeLa cells and from calf spleen also contained NF-kB precursor which could be demonstrated after activation with dissociating agents. These observations strongly suggest that NF-kB is localized as an inactive precursor in the cytosol. Activation of protein kinase C by phorbol ester then would result in two events: induction of DNA-binding activity and nuclear translocation of NF-kB.

Using subcellular fractions from HeLa cells, another TPA-inducible transcription factor, AP-1, was tested to determine whether it also exhibits activation and subcellular redistribution upon TPA-stimulation. As detected by mobility shift assays, AP-1 from nuclear extracts did not show an increase in DNA-binding activity after TPA-stimulation nor were there significant amounts of AP-1 activity present in the cytosolic fractions from control and stimulated cells (Figure 4). This showed that the mechanism by which the transcription factor activity of NF-kB is induced is fundamentally different from that of AP-1 although the initial signal --activation of protein kinase C by phorbol ester - - is the same.

### EXAMPLE 4 Investigation of the DOC-dependence of Cytosolic NF-kB

Cytosol from unstimulated 70Z/3 pre-B cells in buffer A (Dignam, J.P. et al., Nucl. Acids. Res., 11:1475 (1983); Baeuerle, P.A. and D. Baltimore, Cell, 53:211 (1988)) was adjusted to a final concentration of 50mM NaCl, 20mM (HEPES) (pH 7.9), 1.5mM EDTA, 5% glycerol and 0.2% NP-40®. Cytosolic protein (45 mg) was mixed to a final volume of 4 ml with 0.6% DOC, 0.75 g calf thymus (wet weight) DNA-cellulose [Sigma; equilibrated in buffer G: 10 mM Tris-HCl (pH 7.5), 50mM NaCl, 1mM EDTA, 1mM dithiothreitol (DTT), 5% glycerol, 0.2% DOC, 0.2% NP-40®. and 0.5mM phenylmethyl sulfonylfluoride (PMSF)] and 1.2% NP-40. The suspension was incubated in a mini column for 1 hour at room temperature on a rotary shaker. The flow-through fraction was used for gel filtration. DNA-cellulose was washed with buffer G and eluted with a NaCl step gradient in buffer G. Equal proportions of fractions were assayed by EMSA (Sen, R. and D. Baltimore, Cell, 46:705 (1986); Baeuerle, P.A. and D. Baltimore, Cell, 53:211 (1988)) at a final concentration of 1.2% NP-40 in the presence of either 0.03% DOC (non dissociating condition) or 0.6% DOC (dissociating condition) and with 10 µg of bovine serum albumin (BSA) as carrier. Results of this investigation are represented in Figure 10 and described above.

### EXAMPLE 5 Characterization of IkB and its Complex with NF-kB

The flow-through fraction from the DNA-cellulose column (1.55 mg of protein in 250 µl described in Example 4) was subjected to a G-200 Sephadex® column (280 by 7 mm) with a flow rate of 0.15 ml/min in buffer G at room temperature. A mix of size markers (dextran blue; immunoglobulin G, 158 kDa, BSA, 67 kDa, ovalbumin, 45 kDa, myoglobin, 17 kDa; Biorad) was run separately on the column prior to sample runs. Markers were detected in fractions by their color and using SDS-polyacrylamide gel electrophoresis (SDS-PAGE) followed by Coomassie Blue staining.

To detect inhibiting activity, portions of fractions (5 µl; in buffer G) were mixed with 1 µl of nuclear extracts [in buffer D(+)] and 0.5 µl 10% NP-40®. Dignam, J. P. et al., Nucl. Acids Res., 11:1475 (1983). After 30 minutes at room temperature, the reaction volume was brought to 20 µl by the addition of a DNA binding reaction mixture containing 3.2 µg of poly(dI-dC) (Pharmacia), 5 to 20 fmoles of ³²P-end labeled k enhancer fragment, 75mM NaCl, 15mM Tris-HCl (pH 7.5) , 1.5mM EDTA, 1.5mM DTT, 7.5% glycerol, 0.3% NP-40 and 20 µg BSA. After a 20-minute DNA binding reaction, samples were analyzed by EMSA.

Gel filtration fractions containing IkB (25 µg of protein) were incubated for 1 hour at room temperature in buffer G without any addition or with 2 µg of TPCK-treated trypsin (Sigma), 8 µg of BPTI (Sigma), or with 2 µg of trypsin that had been incubated with 8 µg of BPTI. Tryptic digestion was stopped by a 10-minute incubation with 8 µg of BPTI and samples analyzed as described above.

Nuclear extract from TPA-stimulated 70Z/3 cells and cytosol from untreated cells (both 220 µg of protein) were sedimented through 5 ml of a continuous 10 to 30% glycerol gradient in buffer D(+) and 150,000g (SW 50.1 rotor; Beckman) for 20 hours at 4°C. Cosedimented size markers were detected in fractions by SDS-PAGE and Coomassie Blue staining. Portions of glycerol gradient fractions (4 µl) were analyzed by EMSA with 10 µg of BSA as carrier and 0.5 µg of poly(dI-dC). NF-kB precursor was activated by treating 4 µl of fractions with 1.5 µl of formamide before the DNA binding reaction mixture was added.

### EXAMPLE 6 Demonstration of the Presence of the NF-kB--IkB Complex in Enucleated Cells

HeLa cells were grown in Eagle's Minimum Essential Medium supplemented with 10% horse serum, penicillin (50 I.U./ml) and streptomycin (50 µpg/ml) (referred to as MEM-medium) on discs (1.8 cm in diameter) cut from cell culture plastic ware. For enucleation, discs were placed upside down into centrifuge tubes filled with 10 ml of MEM-medium of 37°C containing cytochalasin B (10 µg/ml) and held for the same time in the incubator. To estimate the enucleation efficiency, enucleated cells on one disc were fixed with formaldehyde (3.7%) in phosphate-buffered saline (PBS) for 20 minutes, stained for 4 minutes with 4',6-diamidino-2-phenylindole (DAPI, 1 µg/ml; Sigma) in PBS, and washed in PBS. Fluorescence microscopy under UV light and phase contrast microscopy were performed with a Zeiss Photomicroscope III. Control and enucleated cells were allowed to recover in cytochalasin B-free MEM-medium for 30 minutes before a 2-hour incubation in the absence or presence of TPA (50 ng/ml). Cells were then washed in ice-cold PBS, scraped off the discs in 100 µl of a buffer containing 20mM HEPES (pH 7.9), 0.35M NaCl, 20% glycerol, 1% NP-40® 1mM MgCl₂, 1mM DTT, 0.5mM EDTA, 0.1mM EGTA, 1% aprotinin (Sigma) and 1mM PMSF. After lysis and extraction for 10 minutes on ice, particulate material was removed by centrifugation (Microfuge) for 15 minutes at 4^{o}C and the resulting supernatants were analyzed by EMSA.

### Equivalents

Those skilled in the art will recognize, or be able to ascertain using no more than routine experimentation, many equivalents to the specific embodiments of the invention described specifically herein. Such equivalents are intended to be encompassed in the scope of the following claims.

## Claims

1. A method of altering expression in a cell of a gene whose transcriptional activity is altered by binding of nuclear factor kappa B (NF-κB) to the enhancer of said gene, comprising introducing an agent which controls dissociation of the nuclear factor kappa B-inhibitor of nuclear factor kappa B (NF-κB--IκB) complex present in the cytoplasm of said cell.

2. A method of reducing expression in a cell of a gene whose transcriptional activity is activated by binding of NF-κB to the enhancer of said gene, Comprising introducing an agent which prevents dissociation of NF-κB--IκB complex present in the cytoplasm of said cell.

3. A method of activating in a host cell an NF-κB precursor present in the cytoplasm of said host cell, the precursor comprising an NF-κB--IκB complex, comprising contacting the host cell with an agent capable of causing dissociation of the complex into inhibitor of nuclear factor kappa B (IκB) and NF-κB and translocation of said NF-κB into the nucleus of said cell.

4. A method of claim 3 wherein the said agent is sodium desoxycholate or 12-O-tetradecanoylphorbol 13-acetate.

5. A method of controlling expression of human immunodeficiency virus DNA in a host cell latently infected with human immunodeficiency virus DNA, comprising preventing binding of NF-κB to human immunodeficiency virus transcriptional control elements.

6. A method of claim 5 wherein binding of NF-κB to immunodeficiency virus transcriptional control elements is prevented by inhibiting dissociation of an NF-κB--IκB complex present in the cytoplasm of said host cell into IκB and NF-κB.

7. A method of claim 1 wherein the gene encodes immunoglobulin kappa light chain.

8. A complex of NF-κB and IκB having the characteristics of NF-κB-IκB complex obtainable from cytosolic fractions of unstimulated pre-B cells.

9. The complex of claim 8 wherein the molecular weight is approximately 120 to 130 KD.

10. The complex of claim 9 wherein the NF-κB-IκB complex is obtainable from unstimulated pre-B cells by gel filtration or sedimentation.

11. The complex of claim 10, wherein the unstimulated pre-B cells are unstimulated 70z/3 pre-B cells.

12. NF-κB-IκB complex obtainable from cytosolic fractions of unstimulated pre-B cells.

13. IκB of approximate molecular weight 60 to 70 KD obtainable from the flow-through fraction of cytosolic fractions of unstimulated pre-B cells.

14. IκB of claim 13 obtainable further fractionation of said flow-through fraction of cytosolic fractions and of approximate molecular weight 67 KD.

15. Inactive cytosolic NF-κB precursor which is a heterodimer of an NF-κB molecule of approximate molecular weight 55 to 62 KD and an IκB molecule of approximate molecular weight 60 to 70 KD obtainable by fractionation of unstimulated pre-B cells.

## Patentansprüche

1. Ein Verfahren zum Verhindern der Expression in einer Zelle von einem Gen, dessen Transkriptionsaktivität durch Binden vom Nuklearfaktor kappa B (NF-κB) an den Verstärker dieses Gens verändert ist, das das Einführen eines Mittels umfaßt, das die Dissoziation des Nuklearfaktor kappa B-Inhibitors vom Nuklearfaktor kappa B (NF-κB--IκB) Komplex steuert, der in dem Cytoplasma dieser Zelle vorhanden ist.

2. Ein Verfahren zum Reduzieren der Expression in einer Zelle von einem Gen, dessen Transkriptionsaktivität durch Binden von NF-κB an den Verstärker dieses Gens aktiviert wird, das das Einführen eines Mittels umfaßt, das Dissoziation vom NF-κB--IκB Komplex verhindert, der in dem Cytoplasma dieser Zelle vorhanden ist.

3. Ein Verfahren zum Aktivieren in einer Wirtszelle einer NF-κB Vorstufe, die in dem Cytoplasma dieser Wirtszelle vorhanden ist, wobei die Vorstufe einen NF-κB--IκB Komplex umfaßt, das das Kontaktieren der Wirtszelle mit einem Mittel umfaßt, das in der Lage ist, Dissoziation des Komplexes in einen Inhibitor vom Kernfaktor kappa B (IκB) und NF-κB und Translokation dieses NF-κB in den Kern dieser Zelle zu bewirken.

4. Ein Verfahren nach Anspruch 3, bei dem das Mittel Natriumdesoxycholat oder 12-O-Tetradecanoylphorbol-13-acetat ist.

5. Ein Verfahren zum Steuern der Expression von humaner Immundefizienzvirus-DNA in einer Wirtszelle, die latent mit Human-Immundefizienzvirus-DNA infiziert ist, das das Verhindern des Bindens von NF-κB an Human-Immundefizienzvirus-Transkriptionssteuerelemente umfaßt.

6. Ein Verfahren nach Anspruch 5, bei dem das Binden von NF-κB an Immundefizienzvirus-Transkriptionssteuerelemente verhindert wird, indem Dissoziation eines in dem Cytoplasma von der besagten Wirtszelle vorhandenen NF-κB--IκB Komplexes in IκB und NF-κB verhindert wird.

7. Ein Verfahren nach Anspruch 1, bei dem das Gen eine leichte Immunglobulin-kappa-Kette codiert.

8. Ein Komplex von NF-κB und IκB mit den Charakteristiken vom NF-κB-IκB Komplex, der von cytosolischen Fraktionen von unstimulierten Prä-B Zellen erhältlich ist.

9. Der Komplex nach Anspruch 8, bei dem das Molekulargewicht annäherungsweise 120 bis 130 KD ist.

10. Der Komplex nach Anspruch 9, bei dem der NF-κB-IκB Komplex von unstimulierten Prä-B Zellen durch Gel-Filtrieren oder -Sedimentieren erhältlich ist.

11. Der Komplex nach Anspruch 10, bei dem die unstimulierten Prä-B Zellen unstimulierte 70Z/3 Prä-B Zelllen sind.

12. NF-κB-IκB Komplex, der von cytosolischen Fraktionen von unstimulierten Prä-B Zellen erhältlich ist.

13. IκB mit angenähertem Molekulargewicht von 60 bis 70 KD, das von der Durchfluß-Fraktion von cytosolischen Fraktionen von unstimulierten Prä-B Zellen erhältlich ist.

14. IκB nach Anspruch 13, das durch weiteres Fraktionieren von der besagten Durchflußfraktion von cytosolischen Fraktionen erhältlich ist und ein Molekulargewicht von annäherungsweise 67 KD aufweist.

15. Inaktive cytosolische NF-κB Vorstufe, die ein Heterodimer von einem NF-κB Molekül mit einem angenäherten Molekulargewicht von 55 bis 62 KD und einem IκB Molekül mit einem angenäherten Molekulargewicht von 60 bis 70 KD ist, die durch Fraktionierung von unstimulierten Prä-B Zellen erhältlich ist.

## Revendications

1. Procédé de modification de l'expression, dans une cellule, d'un gêne dont l'activité de transcription est modifieé par liason de facteur nucléaire kappa B (NF-κB) à l'amplificateur dudit gène, comprenant l'opération consistant à introduire un agent qui contrôle la dissociation du complexe inhibiteur de facteur nucléaire kappa B/facteur nucléaire kappa B (NF-κB--IκB) présent dans le cytoplasme de ladite cellule.

2. Procédé de réduction de l'expression, dans une cellule, d'un gêne dont l'activité de transcription est activée par liaison de NF-κB à l'amplificateur dudit gène, comprenant 1'opération consistant à introduire un agent qui empêche la dissociation du complexe NF-κB--IκB présent dans le cytoplasme de ladite cellule.

3. Procédé d'activation, dans une cellule hôte, d'un précurseur de NF-κB présent dans le cytoplasme de ladite cellule hôte, le précurseur étant constitue par un complexe NF-κB--IκB, comprenant l' opération consistant a mettre la cellule hôte en contact avec un agent capable de provoquer la dissociation du complexe en inhibiteur de facteur nucléaire kappa B (IκB) et en NF-κB et la translocation dudit NF-κB dans le noyau de ladite cellule.

4. Procédé selon la revendication 3, dans lequel ledit agent est le désoxycholate de sodium ou le 12-O-tetradécanoylphorbol-13-acetate.

5. Procédé de contrôle de l'expression d'ADN du virus d'immunodéficience humaine dans une cellule hôte infectée de façon latente par l'ADN du virus d,immunodéficience humaine, comprenant l'opération consistant à empêcher la liaison de NF-κB aux elements de contrôle de transcription du virus d'immunodéficience humaine.

6. Procédé selon la revendication 5, dans lequel la liaison de NF-κB aux éléments de contrôle de transcription du virus d'immunodéficience humaine est empêchée par inhibition de la dissociation d'un complexe NF-κB--IκB, present dans le cytoplase de ladite cellule hôte, en IκB et en NF-κB.

7. Procédé selon la revendication 1, dans lequel le gène code pour la chaîne légère kappa de l'immunoglobuline.

8. Complexe de NF-κB et de IκB, ayant les caractéristiques du complexe NF-κB--IκB pouvant être obtenu à partir de fractions cytosoliques de prélymphocrtes B non stimulés.

9. Complexe selon la revendication 8, dont le poids moléculaire est d'environ 120 à 130 kd.

10. Complexe selon la revendication 9, ce complexe NF-κB--IκB pouvant être obtenu à partir de prélymphocytes B non stimulés par filtration sur gel ou sédimentation.

11. Complexe selon la revendication 10, les prélymphocytes B non stimilées étant des pré-lymphocytes B 70Z/3 non stimulés.

12. Complexe NF-κB--IκB, pouvant être obtenu à partir de fractions cytosoliques de pre-lymphocytes B non stimulés.

13. IκB ayant un poids moléculaire d'environ 60 à 70 kd, pouvant être obtenu à partir de la fraction passante de fractions cytosoliques de pré-lymphocytes B non stimulés.

14. IκB selon la revendication 13, pouvant être obtenu par un nouveau fractionnement de ladite fraction passante des fractions cytosoliques et ayant un poids moléculaire d'environ 67 kd.

15. Précuseur inactif de NF-κB cytosolique, qui est un hétérodimere d'une molécule de NF-κB d'un poids moléculaire d'environ 55 à 62 kd et d'une molécule d'IκB d'un poids moléculaire d'environ 60 a 70 kd, pouvant être obtenu par fractionnement de pré-lymphocytes B non stimulés.
